(19) Europäisches Patentamt

European Patent Office

Office européen des brevets

(11) **EP 4 247 201 B1**

(12) **EUROPEAN PATENT SPECIFICATION**

(45) Date of publication and mention
of the grant of the patent:
**08.01.2025 Bulletin 2025/02**

(21) Application number: **21830489.7**

(22) Date of filing: **18.11.2021**

(51) International Patent Classification (IPC):
**A41D 13/015** (2006.01)    **A41D 13/05** (2006.01)
**A41D 13/08** (2006.01)

(52) Cooperative Patent Classification (CPC):
**A41D 13/0556; A41D 13/0153;** A41D 13/084

(86) International application number:
**PCT/IB2021/060698**

(87) International publication number:
**WO 2022/107036 (27.05.2022 Gazette 2022/21)**

(54) **SCALED COMPOSITE STRUCTURE, WEARABLE PROTECTIVE DEVICE, CORRESPONDING MANUFACTURING METHOD AND COMPUTER PROGRAM PRODUCT**

SKALIERTE VERBUNDSTRUKTUR, TRAGBARE SCHUTZVORRICHTUNG, ENTSPECHENDES HERSTELLUNGSVERFAHREN UND COMPUTERPROGRAMMPRODUKT

STRUCTURE COMPOSITE MISE À L'ÉCHELLE, DISPOSITIF DE PROTECTION PORTABLE, METHODE DE FABRICATION CORRESPONDANTE ET PRODUIT DE PROGRAMME INFORMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2020 GB 202018186**

(43) Date of publication of application:
**27.09.2023 Bulletin 2023/39**

(73) Proprietor: **NK Technology Limited**
**London**
**EC1A 2BN (GB)**

(72) Inventor: **KERRES, Natalie Carolin**
**1030 Vienna (AT)**

(74) Representative: **Basck Limited**
**9 Hills Road**
**Cambridge CB2 1GE (GB)**

(56) References cited:
WO-A1-2018/224845    WO-A2-01/87432
US-A- 3 867 239    US-A1- 2009 276 943

**Description**

**TECHNICAL FIELD**

The present disclosure relates generally to a scaled composite structure; more specifically, the present disclosure relates to a method for designing and manufacturing a scaled composite structure based on at least one input parameter.

**BACKGROUND**

In general, it is known to use protective structures to protect and support human or animal body parts from damage. For example, in the health care sector, protective structures are designed to support body parts whilst protecting the body parts from injury or damage. However, known protective structures usually have restricted movement, namely the known protective structures are often not sufficiently flexible. The known protective structures, as used in the health care sector, provide constant protection and support of body parts, which is potentially disadvantageous because the protective structures tend to reduce muscle strength in crucial body areas.

US patent document US2009/276943A1 discloses an impact assembly designed to safeguard a wearer, wherein the impact assembly consists of multiple impact parts, wherein each impact part features a main portion, a first connecting portion, and a second connecting portion. These parts are interconnected to form distinct strings of impact parts. The arrangement involves overlapping the second surface of the main portion of some impact parts with the first surface of others, creating a configuration that enhances protective capabilities for the wearer.

Another US patent document US3867239A discloses an enhanced joint construction for butted, scarf, and overlapped joints utilizing platelets mounted on supporting fabrics, membranes, or netting. The construction is allegedly suitable for use in providing improved flexible lightweight body armor, capable of conforming to the wearer's body contours, adjusting to their movements during vigorous physical activities, and incorporating improved overlapped and butted joint constructions mounted on supporting fabrics.

In an international patent document WO 01/87432A2, there is disclosed an appliance for protecting against impact and strain injuries, allowing greater flexibility of use and interchangeability with other devices or garments. The appliance comprises interconnected plates of impact-resistant material aligned along a common backing member, facilitating limited relative movement between the plates. Means are provided for removably mounting the appliance on an article to be worn. The common backing member, optionally elasticated, may include ligaments centrally or in spaced disposition. The interconnected plates may be attached to the backing member with mutual overlapping, and at least two plates may have fixed relative disposition. The backing member may serve as a fastener, being removably attachable to a garment. Additionally, the appliance may be enclosed within a sleeve, either removably or permanently attached to a garment.

Another international patent document WO 2018/224845A1 relates to a fabric for protecting against impact and strain injuries, wherein the fabric includes multiple layers including a body layer, base layer, and optional surface layer. The layers may be affixed using adhesive and cut using laser cutting for precision. The base layer may be stretchable and made of materials like cotton, while the body layer may comprise a foam. The fabric allows for flexibility, color-changing properties, and potential use in medical and sportswear applications. A method for manufacturing the fabric involves laser cutting, potentially screen printing or dyeing, and forming garments with unique properties, such as insulation and protection without excessive thickness. The fabric's malleability allows for unrestricted movement and highlighting body movements, beneficial for various applications including physiotherapy and fitness training.

There has been ongoing research to develop a flexible protective structure that is capable of protecting as well as allowing a range of the motion to occur. The research may develop a protective structure that is made of flexible and thin material for providing impact protection to body parts.

Furthermore, research on animal scales reveals that the animal scales serve not only as protection against predators, but also address environmental challenges such as friction, compression or hyperextension.

One common drawback to the aforementioned known protective structures is that they restrict motion while providing protection.

Therefore, there arises a need to address the aforementioned technical drawbacks in existing technologies in developing protective structures.

**SUMMARY**

The present disclosure seeks to provide an improved scaled composite structure. Moreover, the present disclosure seeks to provide a method for designing and manufacturing the scaled composite structure. An aim of the present disclosure is to provide a solution that overcomes at least partially the problems encountered in prior art.

According to a first aspect, there is provided a scaled composite structure according to claim 1.

The scaled composite structure is of advantage in that the scales provide a mechanical interlocking effect that is able to limit a range of motion of the scaled composite structure. Furthermore, each of the plurality of 3D scales provide mechanical protection against impact damage. According to a given limit of the range of the motion, the size and the shape of each of the plurality of 3D scales is beneficially customized.

Optionally, in the scaled composite structure, the flexible base layer arrangement enables each of the plurality of 3D scales to only rotate around a base plane in a centre point of each of the plurality of 3D scales.

Optionally, in the scaled composite structure, the flexible base layer arrangement operates against a force that is produced when the limit of the range of motion is applied to the scaled composite structure until interlocking of each of the plurality of 3D scales such that the scaled composite structure provides impact protection against produced force through force distribution.

Optionally, in the scaled composite structure, the size and the shape of at least a given scale of the plurality of 3D scales is a function of a location of the given scale within the scaled composite structure.

Optionally, in the scaled composite structure, the plurality of 3D scales change color when a force applied over the scaled composite structure exceeds a threshold value.

Optionally, in the scaled composite structure, a length and a width of each of the plurality of 3D scales are in a range from 0.01 millimetres (mm) to 500 mm.

Optionally, in the scaled composite structure, each of the plurality of 3D scales comprises a body portion and a nose portion. The nose portion may be characterized as a front extension of each of the plurality of 3D scales which overlaps a preceding 3D scale in the scaled composite structure.

Optionally, in the scaled composite structure, each 3D scale is attached to the flexible base layer arrangement via a plurality of teeth provided at a surface of the 3D scale facing towards the flexible base layer arrangement, wherein the plurality of teeth are arranged to penetrate through the flexible base layer arrangement to attach to a base plate.

According to a second aspect, there is provided a wearable protective device according to claim 7.

The wearable protective device is of advantage in that the device prevents hyperextension injuries caused by sudden impact (example: collision or fall), prevents repetitive strain injury (RSI) through repetitive wrong motion, or limits the motion to help rehabilitation processes. The wearable protective device may be flexible in the range of motion and interlocking the motion when the limit of the range of motion is applied to the wearable protective device. The wearable protective device may also protect a body joint or a body part from impact through force distribution.

Optionally, the wearable protective device further comprises at least one of a double-sided adhesive layer, a type of hydrogel adhesive layer, a silicone adhesive layer or a rubber adhesive layer on one side of the wearable protective device or a sleeve that is interlaced with the wearable protective device for attaching to a skin of a wearer.

According to a third aspect, there is provided a method for designing and manufacturing a scaled composite structure according to claim 9.

The method is of advantage in that it enables production of a series of mass-customized and flexible scaled composite structures for precise motion control, based on the at least one input parameter.

Optionally, in the method, the at least one input parameter is selected from at least one of a type of a part to be covered by the scaled composite structure, data associated with a range of motion of the part, physical parameters of the part, and a type of activity to be performed by the part or on the part. The method may be optimized for cost effectiveness, lightweight of the scaled composite structure and resisting potential of the scaled composite structure to applied force.

Optionally, the method further comprises:

- determining, by using the data processing arrangement, an overall size of each of the plurality of 3D scales and a distance between each of the plurality of 3D scales, based on the at least one input parameter;
- tessellating, by using the data processing arrangement, the flexible base layer arrangement when curved to the limit of the range of motion based on the overall size of the each of the plurality of 3D scales and the distance between each of the plurality of 3D scales; and
- arranging, by using the data processing arrangement, the plurality of 3D scales on the flexible base layer arrangement according to a size and the shape of a base unit after tessellating the flexible base layer arrangement when curved to the limit of the range of motion to determine the size and the shape of each of the plurality of 3D scales.

Optionally, the method further comprises estimating a force to be applied to the scaled composite structure based on the at least one input parameter for enabling determining the overall size of each of the plurality of 3D scales and the distance between each of the plurality of 3D scales.

Optionally, the method further comprises determining (i) a thickness of each of the plurality of 3D scales; (ii) a type of the flexible base layer arrangement for connecting each of the plurality of 3D scales; and (iii) a material for manufacturing the plurality of 3D scales, based on estimated force.

Optionally, the method further comprises determining a diameter and a height of teeth like structures in each of the plurality of 3D scales based on a degree of fineness and a thickness of the type of the first layer, wherein the plurality of teeth are configured to penetrate through the flexible base layer arrangement to attach the 3D scales to the flexible base layer arrangement.

Optionally, the method further comprises generating a 3D model of the scaled composite structure based on a flattened flexible base layer arrangement with the plurality of 3D scales for enabling manufacturing of the scaled composite structure.

Optionally, in the method, the size and the shape of at least one a given scale of the plurality of 3D scales are a function of a location of the given scale within the scaled composite structure. The mechanical interlocking effect of the scaled composite structure is controlled through the size and the shape of each of the plurality of 3D scales.

Optionally, the manufacturing of the scaled composite structure comprises

- providing a flexible base layer arrangement;
- generating a bottom layer of at least one scale of the plurality of 3D scale;
- optionally generating a plurality of teeth like structures projecting from the bottom layer;
- adding a first layer above the bottom layer; and
- generating a top layer of the at least one scale of the plurality of 3D

scales on top of the first layer.

Optionally, the manufacturing of the scaled composite structure comprises connecting the plurality of 3D scales together using at least one of: a base plate, living hinges, stiches. Optionally, the plurality of 3D scales are arranged radially or linearly.

Optionally, the plurality of 3D scales are imprinted with one or more sensors.

According to a fourth aspect, there is provided a computer program product according to claim 15.

It will be appreciated that the aforesaid present method is not merely "software for a computer, as such", "methods of doing a mental act, as such", but has a technical effect in that the method includes designing the scaled composite structure, using the data processing arrangement, in a distributed computing architecture and manufacturing the scaled composite structure using a scale forming unit based on a design of the scaled composite structure. The method for designing the scaled composite structure uses at least one of a parametric algorithm or a non-parametric algorithm to address, for example to solve, the technical problem of designing the scaled composite structure. The present disclosure works as a combination of software and hardware for designing and manufacturing the scaled composite structure.

It will be appreciated that features of the present disclosure are susceptible to being combined in various combinations without departing from the scope of the present disclosure as defined by the appended claims.

## BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:

FIG. 1 is a schematic illustration of a system for designing and manufacturing a scaled composite structure, in accordance with an embodiment of the present disclosure;

FIG. 2A is a top view of an exemplary scaled composite structure in a bent position, in accordance with an embodiment of the present disclosure;

FIG. 2B is a side view of a three-dimensional (3D) scale of FIG. 2A that has teeth-like structures in accordance with an embodiment of the present disclosure;

FIG. 3A is a front view of an exemplary three-dimensional (3D) scale, in accordance with an embodiment of the present disclosure;

FIG. 3B is a side view of an exemplary three-dimensional (3D) scale, in accordance with an embodiment of the present disclosure;

FIG. 3C is a top view of an exemplary three-dimensional (3D) scale, in accordance with an embodiment of the present disclosure;

FIG. 4 is an illustration of a network surface of an exemplary three-dimensional (3D) scale's half body portion, in accordance with an embodiment of the present disclosure;

FIG. 5A is a side view of a first three-dimensional (3D) scale and a second 3D scale in a row in a planar surface, in accordance with an embodiment of the present disclosure;

FIG. 5B is a side view of a first three-dimensional (3D) scale and a second 3D scale in a row in a YZ-plane, in accordance with an embodiment of the present disclosure;

FIG. 6 is an illustration of an exemplary curved surface arranged with a plurality of three dimensional (3D) scales, in accordance with an embodiment of the present disclosure;

FIG. 7A is a vector diagram that illustrates a relationship of a first three-dimensional (3D) scale and a second 3D scale in a first position in a YZ-plane, in accordance with an embodiment of the present disclosure;

FIG. 7B is a vector diagram that illustrates a relationship of a first three-dimensional (3D) scale and a second 3D scale in a second position in a YZ-plane, in accordance with an embodiment of the present disclosure;

FIG. 8A is a top view of an exemplary wearable protective device, in accordance with an embodiment of the present disclosure;

FIG. 8B is a side view of a three-dimensional (3D) scale of FIG. 8A that has teeth-like structures in accordance with an embodiment of the present disclosure;

FIG. 9 is an exemplary graphical representation of a prediction of an injury based on information retrieved from one or more sensors embedded in a scaled composite structure, in accordance with an embodiment of the present disclosure;

FIG. 10 is a flowchart illustrating steps of a method for designing and manufacturing a scaled composite structure, in accordance with an embodiment of the present disclosure;

FIG. 11 is a schematic diagram of an exemplary method for designing a scaled composite structure for protecting a body joint, in accordance with an embodiment of the present disclosure;

FIG. 12 is a schematic diagram of an exemplary method for manufacturing a scaled composite structure, in accordance with an embodiment of the present disclosure;

FIGS. 13A-13B are flow charts of an exemplary method for designing and manufacturing a scaled composite structure for protecting a body j oint, in accordance with an embodiment of the present disclosure;

FIG. 14 is an illustration of an exploded view of a distributed computing architecture or a system in accordance with an embodiment of the present disclosure;

FIG. 15A and 15B are perspective views of the plurality of 3D scales connected through a base plate in accordance with an embodiment of the present disclosure;

FIG. 16A, 16B and 16C are perspective views of the plurality of 3D scales connected through stitches in accordance with an embodiment of the present disclosure;

FIG. 17A and 17B are perspective views of the plurality of 3D scales connected through living hinges in accordance with an embodiment of the present disclosure;

FIG. 18A, 18B and 18C are illustrations of exemplary living hinges for connecting plurality of 3D scales in accordance with an embodiment of the present disclosure; and

FIG. 19 is an illustration of an exemplary scaled composite structure in accordance with various embodiments of the present disclosure.

## DETAILED DESCRIPTION OF EMBODIMENTS

The following detailed description illustrates embodiments of the present disclosure and ways in which they can be implemented.

According to a first aspect, there is provided a scaled composite structure according to claim 1.

The advantage of the scaled composite structure is that it provides the mechanical interlocking effect when the limit of the range of the motion is applied to the scaled composite structure. Otherwise, the scaled composite structure is flexible in the range of the motion. Furthermore, the size and the shape of each of the plurality of 3D scales are a function of the mechanical interlocking effect of the scaled composite structure. According to a given limit of the range of the motion, the size and the shape of each of the plurality of 3D scales are customized. The plurality of 3D scales are flexible in one direction and interlock in another direction.

For example, the scaled composite structure may be used as a motion limiting structure for a wearer to prevent hyperextension of any body joint or a body part, thereby the scaled composite structure prevents injuries of the body joint or the body part. According to parameters associated with the wearer such as a body weight, a body height, a type of the body joint or the body part to be protected, a type of activity (example: rehabilitation, sports performance enhancement, protection, hyperextension protection, impact protection), anthropometry, an age, and medical conditions of the wearer (example: osteoarthritis, carpal tunnel syndrome, etc.), the size and the shape of each of the plurality of 3D scales are controlled to provide the mechanical interlocking effect when the limit of the range of the motion is reached by the body joint or the body part.

Furthermore, the scaled composite structure provides protection to the body joint or the body part without reducing muscle strength of the wearer. The scaled composite structure provides protection to the body j oint or the body part when the limit of the range of the motion is reached by the body joint or the body part. Otherwise, the scaled composite structure remains flexible in the range of the motion. The range of the motion may represent a healthy range of motion of the body joint or the body part.

The scaled composite structure may prevent hyperextension injuries caused by sudden impact (example: collision or fall), repetitive strain injury (RSI) through repetitive wrong motion, or limits the motion to help a rehabilitation process.

The scaled composite structure may be used to protect cables from damage or prevent over-flexing of the cables. The scaled composite structure may be used to protect cables that have application in robotics and automobiles. The cables may be armoured cables. The scaled composite structure may be used as a firefighting cloth or a bullet-proof vest.

The flexible base layer arrangement includes a first layer that may be at least one of a fabric or flexible layer; for example, the fabric is a woven fabric or a unitary layer with perforations. A layer of printed plastics material, for example Nylon®, polyamide material, polypropylene material or similar, may be added between a bottom layer and a top layer of each of the plurality of 3D scales. Alternatively, the fabric or flexible layer can be positioned beneath the bottom layer of each of the plurality of 3D scales which may be connected to the top layer. Alternatively, the fabric or flexible layer can be positioned above the top layer of each of the plurality of 3D scales which may be connected to the bottom layer.

The thickness of the first layer may be in a range of 0.05 mm to 2 mm, more optionally in a range of 0.1 mm to 1mm, namely greater than or equal to 0.1 millimetre (mm) for example. The thickness of the first layer may be equivalent to one layer of a 3D scale.

Optionally, the flexible base layer arrangement has a Young's Modulus in the range from 0.2 to 10 Megapascals (MPa), and has a tensile strength in the range from 0.5 to 20 MPa. In a layman's terms, the flexible base layer arrangement has a Young's modulus and tensile strength generally similar to a woven fabric used for clothing articles.

Optionally, in the scaled composite structure, the flexible base layer arrangement enables each of the plurality of 3D scales to only rotate around a base plane in a centre point of each of the plurality of 3D scales.

Optionally, in the scaled composite structure, the flexible base layer arrangement operates against a force that is produced when the limit of the range of motion is applied to the scaled composite structure until interlocking of each of the plurality of 3D scales such that the scaled composite structure provides impact protection against produced force through force distribution.

When the range of motion is applied to the scaled composite structure, the force may be created in each of the plurality of 3D scales. Thus, it leads to substantial force distribution, not only on the scaled composite structure itself but also on the flexible base layer arrangement that is connecting each of the plurality of 3D scales. During the range of motion, the flexible base layer arrangement operates against created force by enabling each of the plurality of 3D scales to only rotate in the centre point of each of the plurality of 3D scales, thereby the scaled composite structure provides the impact protection against the force through force distribution. The scaled composite structure may provide protection against collision and friction. Moreover, optionally, the plurality of 3D scales are arranged radially or linearly. It will be appreciated that a radially arranged plurality of 3D scales enables better force distribution in comparison to a linearly arranged plurality of 3D scales. Therefore, the radially arranged plurality of 3D scales may be useful on uneven or surfaces that are rounded or not flat. In an example, the radially arranged plurality of 3D scales may be used in applications, such as kneepads, elbow pads, and the like.

Optionally, in the scaled composite structure, the size and the shape of at least a given scale of the plurality of 3D scales is a function of a location of the given scale within the scaled composite structure. For example, a length of a 3D scale positioned on a flat area is higher than to a length of a 3D scale that is positioned on a curved area. Moreover, the radially arranged plurality of 3D scales in the scaled composite structure may control motion in 2 axes, such as angle of rotation in x-y plane. It will be appreciated that for the plurality of 3D scales to be able to control angle of rotation, the plurality of 3D scales is arranged on (namely, attached to) a flexible base layer arrangement, rather than printed flat.

In an embodiment, at least a portion of the plurality of 3D scales in the scaled composite structure are mutually different in the size, and the shape. The at least a portion of the plurality of 3D scales in the scaled composite structure may have an identical size and shape.

Optionally, in the scaled composite structure, the plurality of 3D scales change colour (US: "color") when a force applied over the scaled composite structure exceeds a threshold value.

For example, the plurality of 3D scales change color when the body joint or the body part is injured. The scaled composite structure may provide feedback to the wearer by changing the color of the plurality of 3D scales when the wearer met an accident or the force applied over the scaled composite structure exceeds the threshold value.

Optionally, in the scaled composite structure, a length and a width of the each of the plurality of 3D scales are in a range from 0.01 millimetres (mm) to 500 mm.

Optionally, in the scaled composite structure, each of the plurality of 3D scales comprises a body portion and a nose portion. The nose portion may be characterized as a front extension of each of the plurality of 3D scales which overlaps a preceding 3D scale in the scaled composite structure. A length of the nose portion of the plurality of 3D scales may determine the range of motion on which each of the plurality of 3D scales to rotate until the plurality of 3D scales interlock.

In an embodiment, a material that is used for producing the plurality of 3D scales is selected from a group comprising any resin, carbon, carbon fibre, aramid fibre, Polylactic acid (PLA) polyester, non-Newtonian silicon, Nylon, Polypropylene, Polycarbonate, Thermoplastic Polyurethane (TPU), Polyamide 11 (PA11), Polyamide 12 (PA12), steel, aluminum, elastomeric polyurethane (EPU 40), Rigid Polyurethane 70 (RPU 70), Urethane Methacrylate (UMA90), Acrylonitrile butadiene styrene (ABS), Polyvinyl Alcohol (PVA), Polycarbonate like Translucent, acrylate-based plastic, and ultra-tough white plastic.

Optionally, the material for producing the plurality of 3D scales is selected from a group comprising of polyetheretherketone (PEEK), polyurethane (PU), ethylene vinyl acetate (EVA), polystyrene (PS), styrene acrylonitrile (SAN), acrylonitrile styrene acrylate (ASA), polyethylene terephthalate (PET), polymethyl methacrylate (PMMA), polytetrafluoroethylene (PTFE), Polyoxymethylene (POM), thermoplastic elastomer (TPE), rubber, plastic. Optionally, the material for producing the plurality of 3D scales includes ceramic.

In an embodiment, the first layer is selected from a group comprising of an aramid fibre, carbon fibre, glass fibre, hemp fibre, Nylon and polymer fibre.

The scaled composite structure may include one or more sensors to monitor the motion of the body joint or the body part for predicting injury that is going to happen. The one or more sensors may sense the injury if it happens to a wearer, to provide feedback to cure the injury. The one or more sensors may include at least one of an accelerator sensor, a gyroscope sensor, a flex sensor, an image sensor, a temperature sensor, a radiation sensor, a proximity sensor, a pressure sensor, an optical sensor, or a position sensor. The one or more sensors may be embedded in the scaled composite structure.

Optionally, the plurality of 3D scales are imprinted with the one or more sensors. In this regard, at least one of the plurality of 3D scales, that does not serve as a sensor or that avoids stress so that the parts are not damaged, is printed with at least one motherboard feature. The at least one motherboard feature has a plurality of split features that are printed on to the surrounding 3D scales. It will be appreciated that the electrical connections between the at least one motherboard feature and the plurality of split features is through conductive threads. Beneficially, the at least one motherboard feature and the plurality of split features are miniaturized arrangements that can be freely bent, wound, folded, moved, stretched and dynamically arranged in a three-dimensional space according to various space layout requirements. Additionally, the at least one motherboard feature and the plurality of split features can greatly reduce the volume and the weight of the scaled composite structure. In an embodiment, the at least one motherboard feature is implemented as a flexible printed circuit board (PCB).

According to a second aspect, there is provided a wearable protective device according to claim 7.

The advantage of the wearable protective device is that it provides the mechanical interlocking effect when the limit of the range of the motion is applied to the wearable protective device. Furthermore, the size and the shape of each of the plurality of 3D scales that constitute the wearable protective device are a function of the mechanical interlocking effect of the wearable protective device. According to a given limit of the range of the motion, the size and the shape of each of the plurality of 3D scales is customized.

The wearable protective device may be used to prevent hyperextension of a body joint or a body part, thereby the wearable protective device prevents injuries of the body joint or the body part due to hyperextension.

The wearable protective device may also provide impact protection to a wearer. The wearable protective device may be flexible in the range of the motion.

Optionally, the wearable protective device further includes at least one of a double-sided adhesive layer, a type of hydrogel adhesive layer, a silicone adhesive layer or a rubber adhesive layer on one side of the wearable protective device or a sleeve that is interlaced with the wearable protective device for attaching to a skin of a wearer.

The wearable protective device may be used to prevent hyperextension injuries caused by sudden impact (example: collision or fall), repetitive strain injury (RSI) through repetitive wrong motion, or limit the motion to help rehabilitation process. The wearable protective device may be flexible and protect the body joint or the body part from impact.

According to a third aspect, there is provided a method for designing and manufacturing a scaled composite structure according to claim 9.

The method is of advantage in that it enables production of a series of mass-customized and flexible scaled composite structures for precise motion control, based on the at least one input parameter. The method includes controlling the mechanical interlocking effect of the scaled composite structure through the size and a shape of each of the plurality of 3D scales such that the plurality of 3D scales are overlapping when the scaled composite structure is placed on a planar surface and are intersecting with each other when the limit of the range of motion is applied to the scaled composite structure.

In an embodiment, the data processing arrangement employs at least one of a parametric algorithm or a non-parametric algorithm to design the size and shape of the each of the plurality of 3D scales based on the at least one input parameter. The parametric algorithm or the non-parametric algorithm may be a machine learning algorithm, a regression algorithm, an artificial intelligence (AI) algorithm, or a neural network algorithm.

The method may be used to design and manufacture the scaled composite structure for protecting a body joint or a body part. The method may be used to design and manufacture a firefighting cloth or a bullet-proof vest. The method may be used to design and manufacture the scaled composite structure for protecting cables.

In an embodiment, a shape of the flexible base layer arrangement is determined at a limit of its requirement movement, using the data processing arrangement, based on a two-dimensional curve that is determined based on the limit of the range of motion, a length of the scaled composite structure needed and a width of the scaled composite structure needed.

In an embodiment, the two-dimensional curve is determined using at least one of an image processing model, an artificial intelligence (AI) model, an anthropometry statistics-based model, a 3D camera or a depth camera based on the at least one input parameter. The two-dimensional curve is determined, based on an image of a part to be covered in the limit of the range of the motion, using the at least one of the image processing model, the artificial intelligence (AI) model, the anthropometry statistics-based model, the 3D camera or the depth camera. The image of the part may be a three-dimensional (3D) image or a two-dimensional (2D) image.

The flexible base layer arrangement represents the limit of the range of the motion and covers at least a given area of a part. The part may be the body part or the body joint. The limit of the range of the motion may be a maximum degree of the motion.

A shape of a base unit may include at least one of a rectangular shape, a diamond shape, a rhombus shape, a square shape, a hexagon shape, or a circular shape. The shape of the base unit represents the base shape of each of the plurality of 3D scales.

Optionally, in the method, the at least one input parameter is selected from at least one of a type of a part to be covered by the scaled composite structure, data associated with a range of motion of the part, physical parameters of the part, and a type of activity to be performed by the part or on the part.

In an embodiment, the data associated with the range of motion of the part may include at least one of the image of part to be covered in the limit of the range of motion, or a manual input that includes the limit of the range of motion.

The at least one input parameter may be selected from a type of the body joint or the body part to be protected, an image of the body joint or the body part in the limit of the range of the motion, the limit of the range of the motion, a length and a width of the scaled composite structure needed to cover the body joint or the body part, a body weight, a body height, the type of activity to be performed by a wearer (rehabilitation, sports performance enhancement, protection, hyperextension protection, impact protection), maximum weight of the scaled composite structure, local anthropometry, an age of the wearer, medical conditions of the wearer (osteoarthritis, carpal tunnel syndrome, etc.), color preference of the scaled composite structure, and a choice of whether the scaled composite structure will be worn over or under clothing.

Optionally, the method further includes:

- determining, by using the data processing arrangement, an overall size of each of the plurality of 3D scales and a distance between each of the plurality of 3D scales, based on the at least one input parameter;

- tessellating, by using the data processing arrangement, the flexible base layer arrangement when curved to the limit of the range of motion based on the overall size of the each of the plurality of 3D scales and the distance between each of the plurality of 3D scales; and

- arranging, by using the data processing arrangement, the plurality of 3D scales on the flexible base layer arrangement according to a size and the shape of the base unit after tessellating the flexible base layer arrangement when curved to the limit of the range of motion to determine the size and the shape of each of the plurality of 3D scales.

A centre point of each base unit in the tessellated flexible base layer arrangement when curved to the limit of the range of motion may represent a centre point of each of the plurality of the 3D scale's base.

In an embodiment, arranging the plurality of 3D scales on the flexible base layer arrangement when curved to the limit of the range of motion includes moving a body portion of each of the plurality of 3D scales to the centre point of each base unit. Normal vectors in the centre point of each base unit may be used to choose a direction of each of the plurality of 3D scales. A length of the normal vectors describes a size of the body portion of the plurality of 3D scales. Only the body portion of the plurality of 3D scales may be moved on the centre point of each base unit and, in a next step, the length of each of the plurality of 3D scales are determined to define a nose portion of each of the plurality of 3D scales.

Optionally, the method further includes estimating a force to be applied to the scaled composite structure based on the at least one input parameter for enabling determining the overall size of each of the plurality of 3D scales and the distance between each of the plurality of 3D scales.

The method may include further estimating the force to be applied to the scaled composite structure, based on the physical parameters of the part, and the type of activity to be performed by the part or on the part. The method may include estimating the force to be applied to the scaled composite structure by the wearer or the body joint or body part, based on the body weight, the body height, and the type of activity such as the rehabilitation, the sports performance enhancement, the protection, the hyperextension protection, and the impact protection.

In an embodiment, the method further includes determining a density of the scale composite structure, a base size of each of the plurality of 3D scales, based on an estimated force to be applied by the part or the wearer. The method may include further determining the distance between each of the plurality of 3D scales based on the density of the scale composite structure. The method may include further determining the overall size of each of the plurality of 3D scales based on the base size of each of the plurality of 3D scales.

Optionally, the method further comprises determining (i) a thickness of each of the plurality of 3D scales; (ii) a type of a first layer of the flexible base layer arrangement for connecting each of the plurality of 3D scales; and (iii) a material for manufacturing the plurality of 3D scales, based on estimated force.

Optionally, the method further comprises determining a diameter and a height of teeth-like structures in each of the plurality of 3D scales based on a degree of fineness and a thickness of the type of the flexible base layer arrangement, wherein the teeth-like structures are configured to penetrate through the flexible base layer arrangement to attach the 3D scales thereto.

The method may include determining a degree of fineness of the flexible base layer arrangement and a thickness of the flexible base layer arrangement, based on the type of the flexible base layer arrangement.

According to the invention, the method further includes determining an intersection point between each two scales of the plurality of 3D scales in a row of 3D scales disposed on the flexible base layer arrangement when curved to the limit of the range of motion, to determine the length of each of the plurality of 3D scales in the flexible base layer arrangement.

The intersection point may be calculated using a following equation (1)

$$y = (h - a_1 \times \sin \alpha - a_2 \times \cos \alpha) \times (\cos \alpha\ (a_1 - b_1) + \sin \alpha\ (h - a_2)$$

$$(\sin \alpha\ (a_1 - b_1) + \cos \alpha\ (a_2. - h)$$

$$+(a_1 \times \cos \alpha - a_2. \times \sin \alpha) \hspace{4cm} equation\ (1)$$

Optionally, the method further includes generating a 3D model of the scaled composite structure based on a flattened flexible base layer arrangement with the plurality of 3D scales for enabling manufacturing of the scaled composite structure. During the flattening process, the tessellated flexible base layer arrangement arranged with the plurality of 3D scales, a location, and size and shape of each of the plurality of 3D scales remain constant. Each of the plurality of 3D scales do not touch each other in the planar surface.

In an embodiment, the method further includes developing a geometric code (GCODE) based on the 3D model of the scaled composite structure for enabling manufacturing of the scaled composite structure.

Optionally, in the method, the size and the shape of at least one a given scale of the plurality of 3D scales are a function of a location of the given scale within the scaled composite structure. The mechanical interlocking effect of the scaled composite structure is controlled through the size and the shape of each of the plurality of 3D scales.

Optionally, the manufacturing of the scaled composite structure includes

- providing a flexible base layer arrangement;

- generating a bottom layer of at least one scale of the plurality of 3D scales;

- optionally generating a plurality of teeth like structures projecting from the bottom layer;

- adding a first layer above the bottom layer; and

- generating a top layer of the at least one scale of the plurality of 3D scales on top of the first layer.

In an embodiment, the manufacturing of the scaled composite structure includes

- moulding the top layer of the at least one scale of the plurality of 3D scales along with or without the plurality of teeth like structures as a one-step moulding process;

- adding the first layer below the top layer, wherein the teeth like structures optionally penetrate through the first layer; and

- connecting the bottom layer to the top layer by 3D printing or by thermal fuse welding.

The bottom layer and the top layer of the at least one scale of the plurality of 3D scales are optionally connected with a one-way pin system when moulded parts of the bottom layer and the top layer are put together.

The one-step moulding process may include at least one of an injection moulding, or an over-moulding. A method of manufacturing the scaled composite structure includes at least one of an additive manufacturing or 3D printing, or moulding.

In an embodiment, the method includes positioning the first layer in between the bottom layer and the top layer of at least one scale of the plurality of 3D scales. Alternatively, the first layer can be positioned beneath the bottom layer which may be connected to the top layer. Alternatively, the first layer can be positioned above the top layer which may be connected to the bottom layer.

In an embodiment, the method further includes attaching the scaled composite structure onto a skin of the wearer through at least one of: a double-sided adhesive, a type of hydrogel adhesive layer, a silicone adhesive layer or a rubber adhesive layer included on the scaled composite structure, or a sleeve that is interlaced with the scaled composite structure.

In an embodiment, the method further includes monitoring, using one or more sensors, the part covered with the scaled composite structure, and predicting the injury to the part covered with the scaled composite structure based on the data retrieved from the one or more sensors. The one or more sensors may be embedded in the scaled composite structure. The one or more sensors may include at least one of an accelerator sensor, a gyroscope sensor, a flex sensor, an image sensor, a temperature sensor, a radiation sensor, a proximity sensor, a pressure sensor, an optical sensor, or a position sensor.

The method may include further providing feedback to cure injury of the part if the injury happens to the part, based on the data retrieved from the one or more sensors.

The method for designing and manufacturing the scaled composite structure for protecting the body part may include (i) receiving at least one input parameter that includes data associated with the body part to be protected, the body specifications of the wearer, and the type of activity to be performed by the wearer, (ii) determining a flexible base layer arrangement that represents the limit of the motion and covers at least a portion of the body part and a shape of the flexible base layer arrangement, based on the data associated with the body part to be protected, (iii) determining an overall size of each of a plurality of 3D scales to be used for producing the scaled composite structure and a distance between each of the plurality of 3D scales, based on the body specifications of the wearer, and the type of activity to be performed by the wearer, (iv) tessellating the flexible base layer arrangement when curved to the limit of the range of motion based on the overall size of the each of the plurality of 3D scales and the distance between each of the plurality of 3D scales, (v) arranging the plurality of 3D scales on the tessellated flexible base layer arrangement according to a size and the shape of a base unit, (vi) determining a length of each of the plurality of 3D scales according to a location of each of the plurality of 3D scales in the flexible base layer arrangement to define a size and a shape of each of the plurality of 3D scales arranged on the flexible base layer arrangement, and (vii) generating the plurality of 3D scales that are connected together by the flexible base layer arrangement to produce the scaled composite structure, based on the size and the shape of each of the plurality of 3D scales arranged on the flexible base layer arrangement.

In an example, there is provided a system for designing and manufacturing a scaled composite structure. The system includes a memory that stores a set of instructions, a data processing arrangement that is in communication with the memory, and a scale forming unit. When in operation, the data processing arrangement is configured to execute the set of instructions to perform(i) receiving at least one input parameter from a user, (ii) determining a flexible base layer arrangement and a shape of the flexible base layer arrangement, based on at least one input parameter, at a limit of a range of motion to be applied to the scaled composite structure (iii) determining a size and a shape of each of a plurality of 3D scales that are arranged on the flexible base layer arrangement when curved to the limit of the range of motion, thereby determining a length of each of the plurality of 3D scales, and (iv) enabling the scale forming unit to manufacture the scaled composite structure. The scale forming unit manufactures the scaled composite structure based on the size and the shape of each of the plurality of 3D scales that are arranged on the flexible base layer arrangement when curved to the limit of the range of motion. The data processing arrangement may be communicatively connected with the scale forming unit. The scale forming unit may be a three-dimensional (3D) printer, or a moulding apparatus.

Optionally, the manufacturing of the scaled composite structure comprises connecting the plurality of 3D scales together, wherein the plurality of 3D scales are connected together using at least one of: a base plate, living hinges, or stiches. In this regard, each of the plurality of 3D scales are connected together for allowing a range of motion of the scaled composite structure. Beneficially, connecting each of the plurality of 3D scales together results in a high strength, flexible scaled composite structure.

Optionally, the plurality of 3D scales that are arranged on the flexible base layer arrangement are connected using a base plate method. In the base plate method, a base plate is 3D printed. The base plate comprises a plurality of holes therein to reduce stress points. Subsequently, the plurality of 3D scales are printed on top of the base plate. Optionally, 3D printing of the base plate and the plurality of 3D scales on top of the base plate is done by SLS printing. Notably, SLS printing allows adjusting material quantities during printing. Beneficially, changing material of base plate during printing enables the printing of a stronger, more flexible scaled composite structure. It will be appreciated that while connecting the plurality of 3D scales using the base plate method the 3D printing process is not required to be paused before printing the plurality of 3D scales on top of the base plate.

Optionally, the plurality of 3D scales that are arranged on the flexible base layer arrangement are connected using a living hinges method. In the living hinges method, the plurality of 3D scales are 3D printed together based on the pre-determined size and structure of the wearable protective device. The plurality of 3D scales are connected together though living hinges without requiring a base plate. Beneficially, the use of living hinges results in a stronger, more flexible scaled composite structure. It will be appreciated that while connecting the plurality of 3D scales using the living hinges method the 3D printing process is not required to be paused before printing the plurality of 3D scales and connecting the plurality of 3D scales though living hinges.

Optionally, the plurality of 3D scales that are arranged on the flexible base layer arrangement are connected using a stitch method. More optionally, the plurality of 3D scales are connected using Kevlar stiches. The Kevlar (also called para-aramid) is a heat-resistant and strong synthetic fiber that is typically spun into ropes or fabric sheets that can be used as such, or as an ingredient in composite material components. Herein, the Kevlar stitches are used to bond the base unit of a scale and top part of an adjacent 3D scale. Beneficially, said manufacturing using Kevlar stitches provides better adhesion between the 3D scales and the Kevlar. Moreover, connecting the plurality of 3D scales using Kevlar stiches results in a stronger, more flexible, cheaper and more environmentally friendly scaled composite structure. Additionally, while connecting the plurality of 3D scales using Kevlar stiches, a lesser amount of Kevlar is required. It will be appreciated that while connecting the plurality of 3D scales using Kevlar stiches the 3D printing process is required to be paused while incorporating each Kevlar stitch between the base unit of a scale and the top part of an adjacent 3D scale.

The data processing arrangement and scale forming unit may be located remotely and may work as cohesive units. The data processing arrangement may communicate with the scale forming unit over a network. The network may be a wired network, e.g., fiber optic, Ethernet, Fiber Channel, direct connections, and close-range communications, a wireless network, e.g., Wi-Fi, combination of the wired network and the wireless network, a LAN (local area network), WAN (wide area network), the Internet, cable networks, and cellular networks.

The system may include at least one input interface to receive the at least one input parameter and at least one output interface to provide information about the size and the shape of each of the plurality of 3D scales.

The data processing arrangement may include one or more processors to execute the set of instructions stored in the memory and a database that stores a data driven model to perform one or more functions of the data processing arrangement.

Embodiments of the present disclosure substantially eliminate or at least partially address the aforementioned technical drawbacks in existing technologies in controlling motion by providing the scaled composite structure that interlocks when the limit of the range of the motion is applied to the scaled composite structure, otherwise the scaled composite structure remains flexible.

## DETAILED DESCRIPTION OF THE DRAWINGS

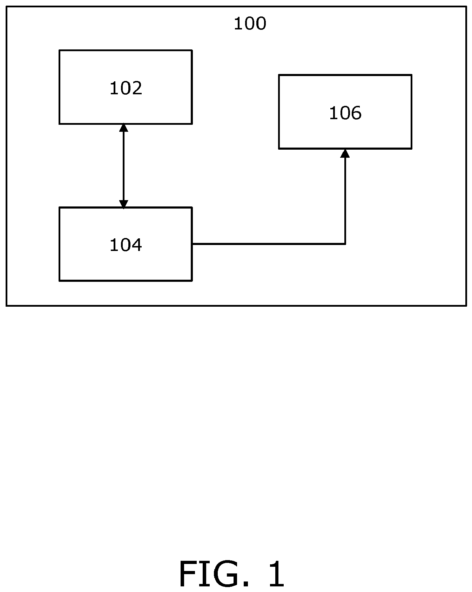

FIG. 1 is a schematic illustration of a system 100 for designing and manufacturing a scaled composite structure, in accordance with an embodiment of the present disclosure. The system 100 includes a memory 102 that stores a set of instructions, a data processing arrangement 104 that is in communication with the memory 102, and a scale forming unit 106. When in operation, the data processing arrangement 104 is configured to execute the set of instructions to perform (i) receiving at least one input parameter from a user or a wearer, (ii) determining a flexible base layer arrangement and a shape of the flexible base layer arrangement, based on at least one input parameter, at a limit of a range of motion to be applied to the scaled composite structure (iii) determining a size and a shape of each of a plurality of 3D scales that are arranged on the flexible base layer arrangement when curved to the limit of the range of motion, thereby determining a length of each of the plurality of 3D scales, and (iv) enabling the scale forming unit 106 to manufacture the scaled composite structure. The scale forming unit 106 manufactures the scaled composite structure based on the size and the shape of each of the plurality of 3D scales that are arranged on the flexible base layer arrangement when curved to the limit of the range of motion. The scaled composite structure provides a mechanical interlocking effect when a limit of a range of motion is applied to the scaled composite structure and is flexible in the range of the motion.

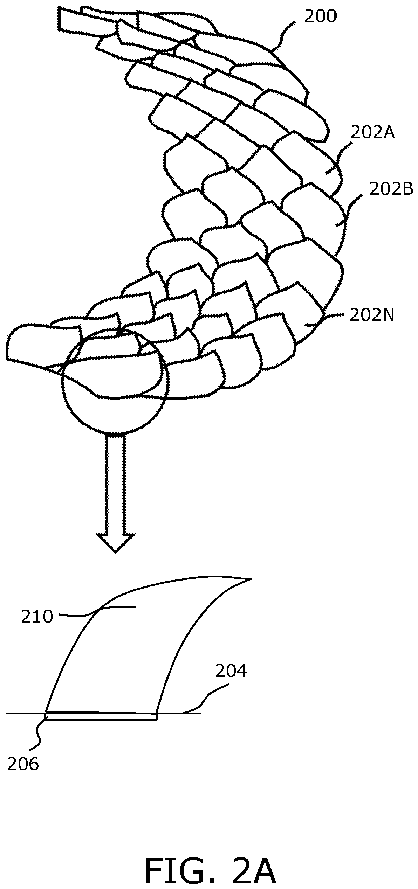

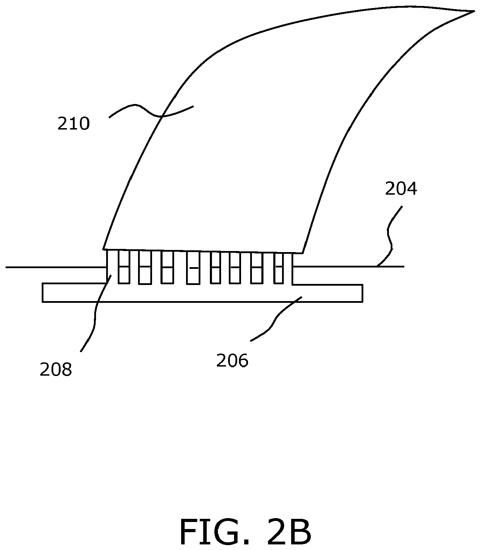

FIG. 2 is a top view of an exemplary scaled composite structure **200** in a bended position, in accordance with an embodiment of the present disclosure. The exemplary scaled composite structure **200** includes a plurality of three dimensional (3D) scales **202A-N** and a flexible base layer arrangement **204.** The plurality of three dimensional (3D) scales **202A-N** are attached to the flexible base layer arrangement **204.** Each of the plurality of 3D scales **202A-N** includes a bottom layer **206** and a top layer **210** that is present on top of the flexible base layer arrangement **204.** The plurality of 3D scales **202A-N** are overlapping when the exemplary scaled composite structure **200** is placed on a planar surface. The plurality of 3D scales **202A-N** are intersecting with each other when a limit of a range of motion is applied to the exemplary scaled composite structure **200,** to provide a mechanical interlocking effect. The flexible base layer arrangement **204** may enable each of the plurality of 3D scales **202A-N** to only rotate around a base plane in a centre point of each of the plurality of 3D scales **202A-N.**

FIG. 2B is a side view of a three-dimensional (3D) scale of FIG. 2A that has a plurality of teeth **208** in accordance with an embodiment of the present disclosure. Each of the plurality of 3D scales **202A-N** includes the bottom layer **206,** a plurality of teeth **208,** and the top layer **210** that is present on top of the plurality of teeth **208.** The row of teeth-like structures **208** may penetrate through the flexible base layer arrangement **204.**

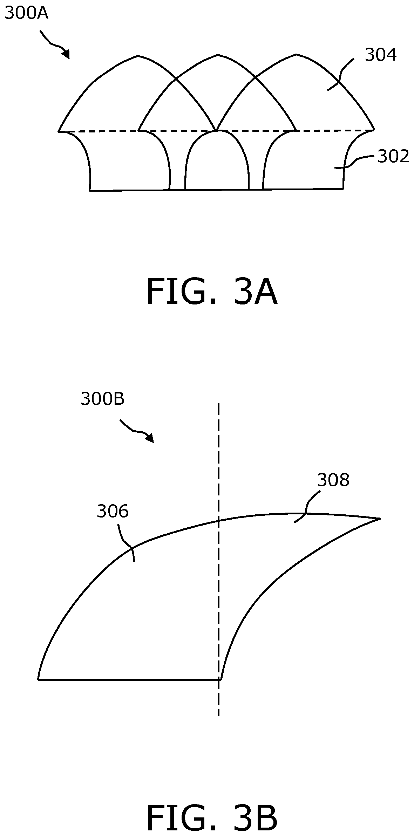

FIG. 3A is a front view of an exemplary three-dimensional (3D) scale **300A,** in accordance with an embodiment of the present disclosure. The exemplary 3D scale **300A** includes a body portion **302** and a nose portion **304.** The body portion **302** is defined through a shape of a base unit of a flexible base layer arrangement that is determined based on at least one input parameter. The nose portion **304** is characterized as a front extension of the exemplary 3D scale **300A** which overlaps a preceding 3D scale in a scaled composite structure. A length of the exemplary 3D scale **300A** within the scaled composite structure may be controlled through given input parameters. Therefore, controlling the length of the exemplary 3D scale **300A** allows the scaled composite structure to interlock when a limit of a range of motion is applied to the scaled composite structure.

FIG. 3B is a side view of an exemplary three-dimensional (3D) scale **300B,** in accordance with an embodiment of the present disclosure. The exemplary 3D scale **300B** includes a body portion **306** and a nose portion **308.** A length of the nose portion **308** of the exemplary 3D scale **300B** may determine how much the exemplary 3D scale **300B** in front may be able to rotate until the exemplary 3D scale **300B** interlock. The length of the nose portion **308** of the exemplary 3D scale **300B** is controlled through given input parameters.

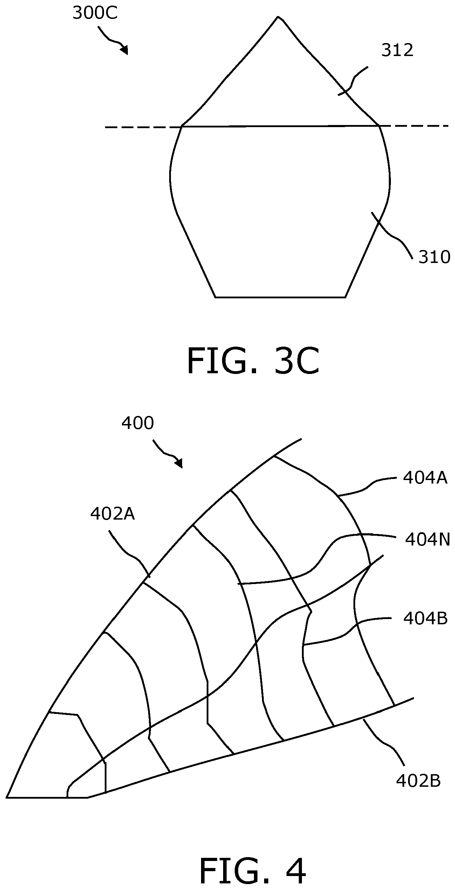

FIG. 3C is a top view of an exemplary three-dimensional (3D) scale **300C,** in accordance with an embodiment of the present disclosure. The exemplary 3D scale **300C** includes a body portion **310** and a nose portion **312.** A length of the nose portion **312** of the exemplary 3D scale **300C** controls flexibility of the exemplary 3D scale **300C** in a scaled composite structure. Therefore, a nose portion of each 3D scale in the scaled composite structure has a specific length, depending on each 3D scale's location on the scaled composite structure.

FIG. 4 is an illustration of a network surface of an exemplary three-dimensional (3D) scale's half body portion **400,** in accordance with an embodiment of the present disclosure. One or more first curves **402A-B** in V direction and one or more second curves **404A-N** in U direction are used to shape the exemplary 3D scale's half body portion **400.**

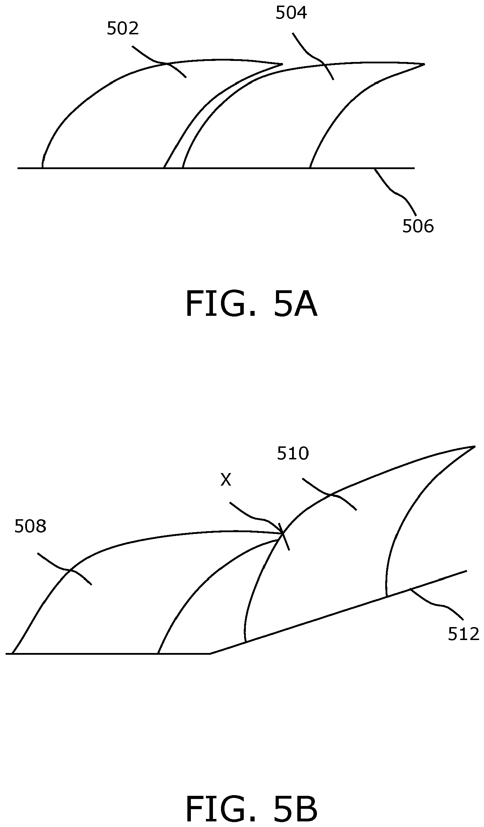

FIG. 5A is a side view of a first three-dimensional (3D) scale **502** and a second 3D scale **504** in a row in a planar surface **506,** in accordance with an embodiment of the present disclosure. The first 3D scale **502** and the second 3D scale **504** do not intersect with each other (as shown in FIG. 5A) when the first 3D scale **502** and the second 3D scale **504** are placed on the planar surface **506.** A base of the first 3D scale **502** and the second 3D scale **504** may be touching with each other. A size and a shape of the first 3D scale **502** and the second 3D scale **504** in a scaled composite structure are controlled through at least one input parameter. The size and the shape of the first 3D scale **502** and the second 3D scale **504** are a function of a location of the first 3D scale **502** and the second 3D scale **504** within the scaled composite structure.

FIG. 5B is a side view of a first three-dimensional (3D) scale **508** and a second 3D scale **510** in a row in a YZ plane **512,** in accordance with an embodiment of the present disclosure. The first 3D scale **508** and the second 3D scale **510** are intersecting at an intersection point **X** (as shown in FIG. 5B) when the first 3D scale **508** and the second 3D scale **510** are placed on the YZ plane **512.** The intersection point **X** may be calculated using a following equation (1):

$$y = \frac{(h - a_1 \times \sin\alpha - a_2 \times \cos\alpha) \times (\cos\alpha\,(a_1 - b_1) + \sin\alpha\,(h - a_2)}{(\sin\alpha\,(a_1 - b_1) + \cos\alpha\,(a_2. - h)}$$

$$+(a_1 \times \cos\alpha - a_2. \times \sin\alpha) \qquad\qquad \text{equation (1)}$$

The equation (1) may be used to determine a size and a shape of the first 3D scale **508** and the second 3D scale **510** within a scaled composite structure. The size and the shape of the first 3D scale **508** and the second 3D scale **510** are a function of a location of the first 3D scale **508** and the second 3D scale **510** within the scaled composite structure.

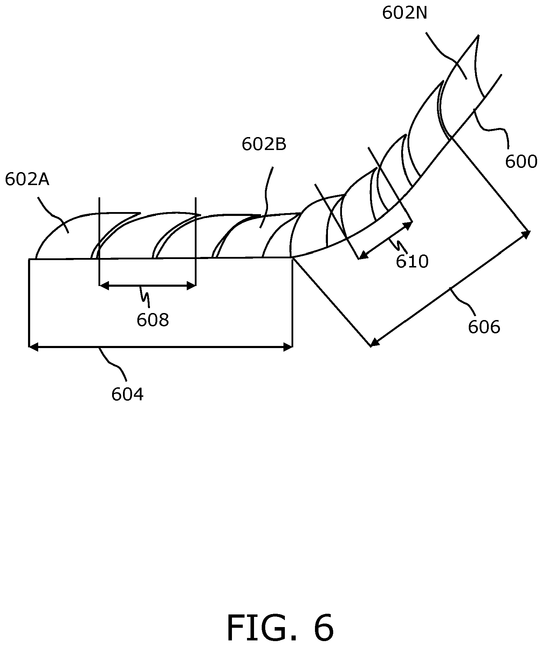

FIG. 6 illustrates an exemplary curved surface **600** arranged with a plurality of three dimensional (3D) scales **602A-N,** in accordance with an embodiment of the present disclosure. The exemplary curved surface **600** is arranged with the plurality of 3D scales **602AN.** The exemplary curved surface **600** includes a flat area **604** and a curved area **606.** Each of the plurality of three dimensional (3D) scales **602A-N** has different length according to its location in the exemplary curved surface **600.** For example, a 3D scale positioned in the flat area **604** has higher length **608** than a 3D scale positioned in the curved area **606.** The 3D scale positioned in the curved area **606** has lower length **610** than the 3D scale positioned in the flat area **604.** To calculate the length of each of the plurality of 3D scales **602A-N,** an intersecting point of each two 3D scales at a rotation angle has to be calculated.

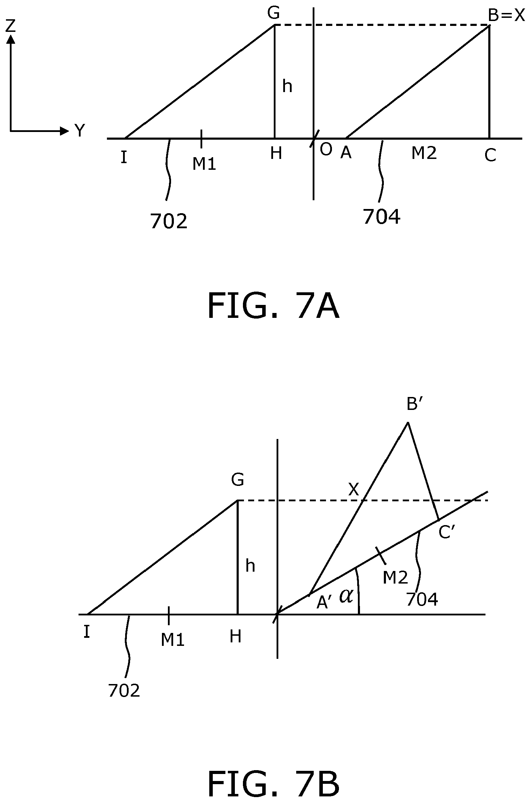

FIG. 7A is a vector diagram that illustrates a relationship of a first three-dimensional (3D) scale **702** and a second 3D scale **704** in a first position in YZ-plane, in accordance with an embodiment of the present disclosure. In FIG. 7A, to simplify a complex shape of an individual 3D scale, triangles represent the individual 3D scale's contour. The first 3D scale **702** and the second 3D scale **704** are placed on a planar surface in the first position. A point **I** represents a starting point of the first 3D scale **702.** A point **H** represents an ending point of the first 3D scale **702.** A point **G** represents a front point of a nose portion of the first 3D scale **702.** The point **G** is parallel to their base as a vector from **I** to **H.** A point M1 represents a center point of the first 3D scale's 702 base shape.

Moreover, h represents a height of the first 3D scale 702. A point A represents a starting point of the second 3D scale **704.** A point **C** represents an ending point of the second 3D scale **704.** A point **B** represents a front point of a nose portion of the second 3D scale **704.** The point **B** is parallel to their base as a vector from **A** to **C.** A point **M2** represents a center point of the second 3D scale's **704** base shape. A point **O** represents a point of rotation between the first 3D scale **702** and the second 3D scale **704,** which is a midpoint between the point **H** and the point **A.** Base planes of the first 3D scale **702** and the second 3D scale **704** are 0 degree rotated to one another. Therefore, a front point **X** is an intersection of the vector *IH* in the point **G** and a line between the points **A** and **B.** In the first position, the intersecting point **X** is equivalent to the point **B.**

FIG. 7B is a vector diagram that illustrates a relationship of a first three-dimensional (3D) scale **702** and a second 3D scale **704** in a second position in YZ-plane, in accordance with an embodiment of the present disclosure. In FIG. 7B, to simplify a complex shape of an individual 3D scale, triangles represent the individual 3D scale's contour. A point **I** represents a starting point of the first 3D scale **702.** A point **H** represents an ending point of the first 3D scale **702.** A point **G** represents a front point of a nose portion of the first 3D scale **702.** A point **M1** represents a center point of the first 3D scale's **702** base shape. **h** represents a height of the first 3D scale **702.** A point **A'** represents a starting point of the second 3D scale **704.** A point **C'** represents an ending point of the second 3D scale **704.** A point **B'** represents a front point of a nose portion of the second 3D scale **704.** A point **M2** represents a center point of the second 3D scale's **704** base shape. A point **O** represents a point of rotation between the first 3D scale **702** and the second 3D scale **704.** In the second position, the first 3D scale **702** and the second 3D scale **704** are rotated around the point **O** with an angle

$\alpha$. A point **X** is an intersection point that moves along vector **AB** in the second Position. As the point **X** also remains on the vector **IH** in the point **G,** the intersection point **X** always has same height h and therefore an equivalent Z-coordinate as the point **G.** Vector calculations in two dimensional are applied to locate a Y-coordinate of the intersection point X ($y$ $z$). A straight line is created through the points **A'** and **B'.** For the straight line, equation y=kx+d, a slope **k** is calculated for the points **A'** and **B'** and consequentially d (intercept on a y-axis). The z-coordinate of the intersection point **X** is deployed. A following final equation (2) uses the points **A** and **B** from the first position (from FIG. 7A) and calculates the y-coordinate of the intersection point in the second position.

$$y = \frac{(h - a_1 \times \sin \alpha - a_2 \times \cos \alpha) \times (\cos \alpha \ (a_1 - b_1) + \sin \alpha \ (h - a_2)}{(\sin \alpha \ (a_1 - b_1) + \cos \alpha \ (a_2. - h)}$$

$$+(a_1 \times \cos \alpha - a_2. \times \sin \alpha) \qquad equation(2)$$

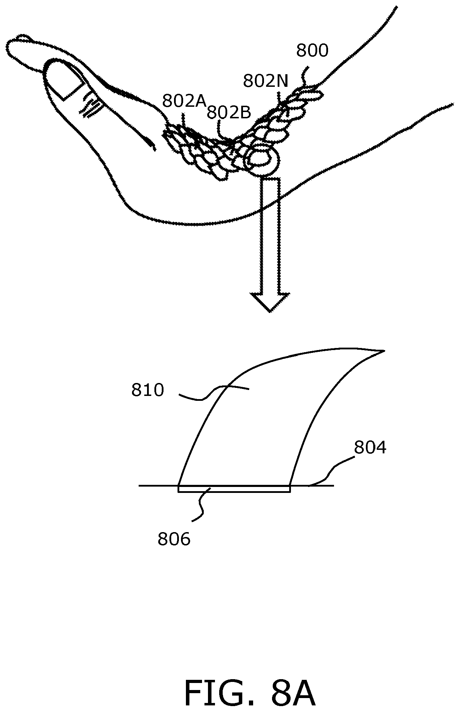

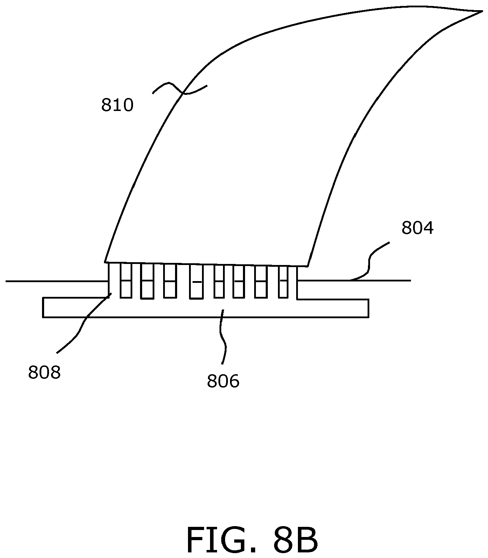

FIG. 8 is a top view of an exemplary wearable protective device **800,** in accordance with an embodiment of the present disclosure. The exemplary wearable protective device **800** includes a plurality of three dimensional (3D) scales **802A-N** and a flexible base layer arrangement **804** that is connecting each of the plurality of 3D scales **802A-N.** Each of the plurality of 3D scales **802A-N** includes a bottom layer **806,** and a top layer **810** that is present on top of the flexible base layer arrangement **204.** The plurality of 3D scales **802A-N** are overlapping when the exemplary wearable protective device **800** is placed on a planar surface. The plurality of 3D scales **802A-N** are intersecting with each other when a limit of a range of motion is applied to the exemplary wearable protective device **800,** to provide a mechanical interlocking effect.

FIG. 8B is a side view of a three-dimensional (3D) scale of FIG. 8A that has a plurality of teeth **808** in accordance with an embodiment of the present disclosure. Each of the plurality of 3D scales **802A-N** includes the bottom layer **806,** the plurality of teeth **808,** and the top layer **810** that is present on top of the plurality of teeth **808.** The plurality of teeth **808** may penetrate through the flexible base layer arrangement **804.**

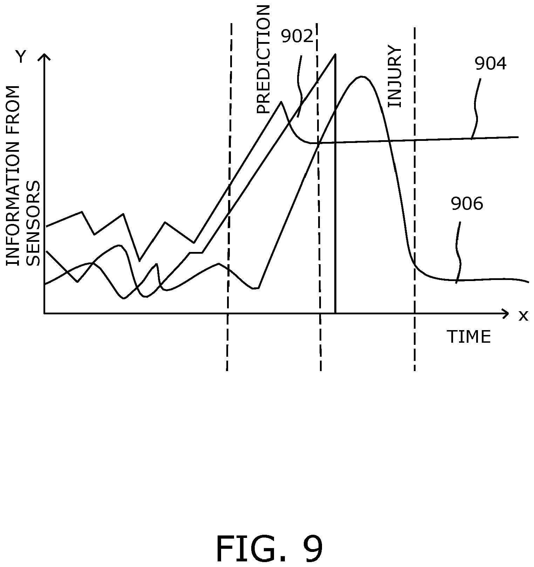

FIG. 9 is an exemplary graphical representation of prediction of an injury based on information retrieved from one or more sensors embedded in a scaled composite structure, in accordance with an embodiment of the present disclosure. In the exemplary graphical representation of the prediction of the injury, in an ordinate Y-axis plotted against time in an abscissa X-axis to information from sensors. A curve **902** represents information retrieved from an accelerator sensor that is embedded in the scaled composite structure, over a period of time. A curve **904** represents information retrieved from a gyroscope sensor that is embedded in the scaled composite structure, over the period of time. A curve **906** represents information retrieved from a flex sensor that is embedded in the scaled composite structure, over the period of time. The exemplary graphical representation shows an injury prediction that is going to happen, for hyperextending wrists based on the information retrieved from one or more sensors embedded in the scaled composite structure.

The one or more sensors in the scaled composite structure may sense the injury if it happens to a body part (example: wrist) and may transfer sensed data to an analytical server to analyse and provide feedback to a user or a wearer to cure the injury.

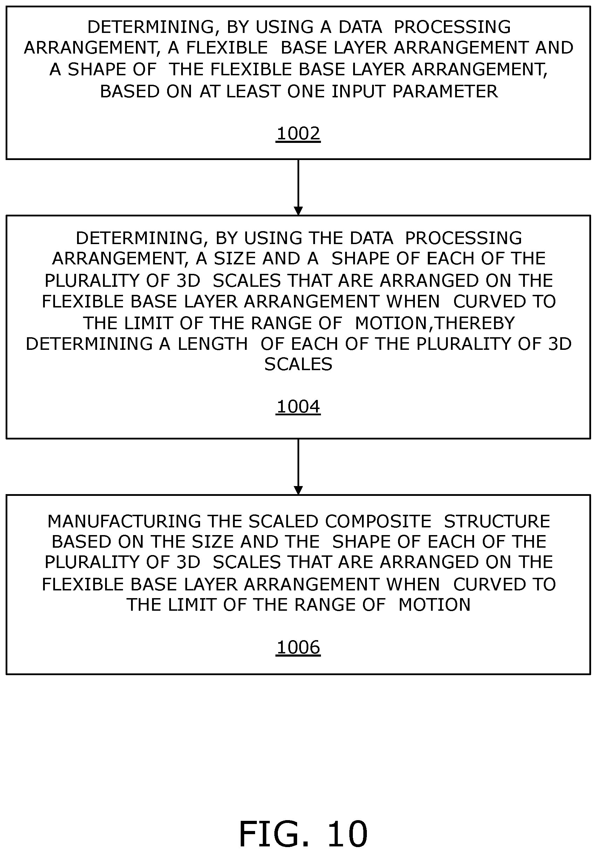

FIG. 10 is a flowchart illustrating steps of a method for designing and manufacturing a scaled composite structure, in accordance with an embodiment of the present disclosure. The scaled composite structure includes a plurality ofthree-dimensional (3D) scales that are overlapping when the scaled composite structure is placed on a planar surface and a first layer that is connecting each of the plurality of 3D scales. At a step **1002,** a flexible base layer arrangement and a shape of a flexible base layer arrangement are determined, using a data processing arrangement, based on at least one input parameter. At a step **1004,** a size and a shape of each of the plurality of 3D scales that are arranged on the flexible base layer arrangement when curved to the limit of the range of motion are determined, using the data processing arrangement, thereby determining a length of each of the plurality of 3D scales. At a step **1006,** the scaled composite structure is manufactured based on the size and the shape of each of the plurality of 3D scales that are arranged on the flexible base layer arrangement when curved to the limit of the range of motion.

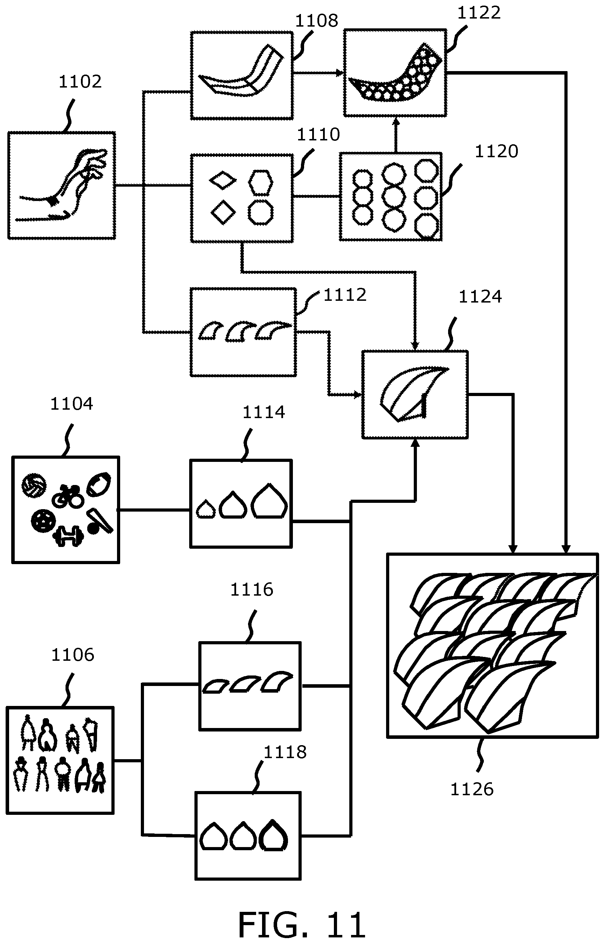

FIG. 11 is a schematic diagram of an exemplary method for designing a scaled composite structure for protecting a body joint, in accordance with an embodiment of the present disclosure. At a step **1102,** data associated with a body joint to be protected is received. The data associated with the body j oint may include an image of the body j oint in a maximum range of motion. Optionally, the data associated with the body joint may include manual input such as a type of the body joint, the maximum range of motion of the body joint, a length and a width of an area to be covered. At a step **1104,** a type of activity to be performed by a wearer is received. At a step **1106,** body specifications of the wearer are received. At a step **1108,** a flexible base layer arrangement is determined based on the data associated with the body joint. At a step **1110,** a shape of the flexible base layer arrangement is determined based on the data associated with the body j oint. At a step **1112,** a length of each of a plurality of 3D scales is determined based on the data associated with the body j oint. At a step **1114,** a size of each of the plurality of 3D scales is determined based on the type of activity to be performed by the wearer. At a step **1116,** a height of each of the plurality of 3D scales is determined based on the body specifications of the wearer. At a step **1118,** a thickness of each of the plurality of 3D scales is determined based on the body specifications of the wearer. At a step **1120,** a tessellation density is determined based on a shape of a flexible base layer arrangement. At a step **1122,** the flexible base layer arrangement when curved to the limit of the range of motion is tessellated into the shape of the base unit based on the tessellation density. At a step **1124,** a geometry of each of the plurality of 3D scales is determined based on the length, the size, the height, and the thickness of the each of the plurality of 3D scales. At a step **1126,** the scaled composite structure is designed based on tessellated flexible base layer arrangement and the geometry of each of the plurality of 3D scales.

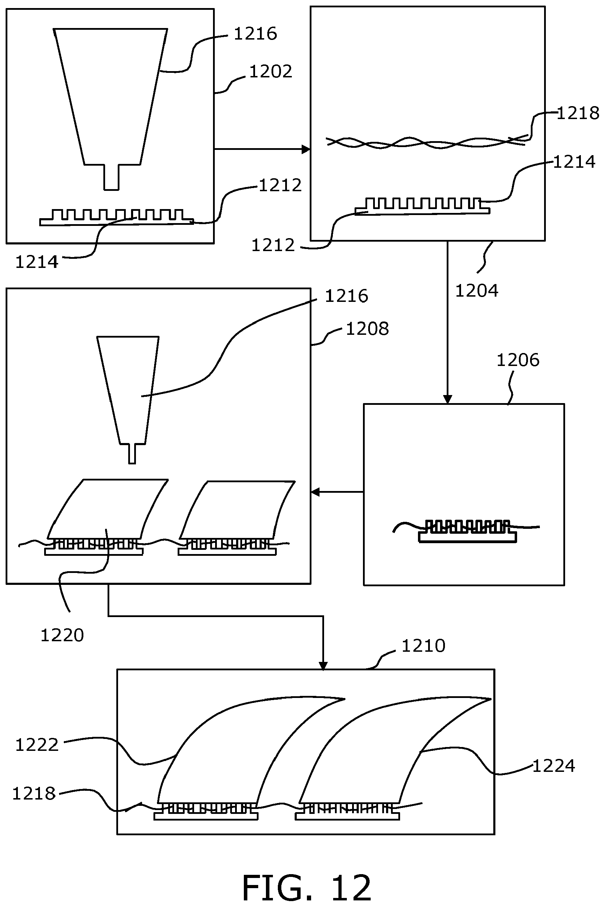

FIG. 12 is a schematic diagram of an exemplary method for manufacturing a scaled composite structure, in accordance with an embodiment of the present disclosure. At a step **1202,** a base layer **1212** of a three-dimensional (3D) scale is printed using a 3D printer **1216,** thereafter a row of teeth-like structures **1214** is printed on top of the base layer **1212** using the 3D printer **1216.** At a step **1204,** a first layer **1218** is placed on top of the row of teeth-like structures **1214.** At a step **1206,** the first layer **1218** is pushed through the plurality of teeth like structures **1214.** At a step **1208,** a top layer **1220** of the 3D scale is printed, using the 3D printer **1216,** on top of the row of teeth-like structures **1214.** At a step **1210,** a first 3D scale **1222** and a second 3D scale **1224** are manufactured in a row using the 3D printer **1216,** that are connected by the first layer **1218.**

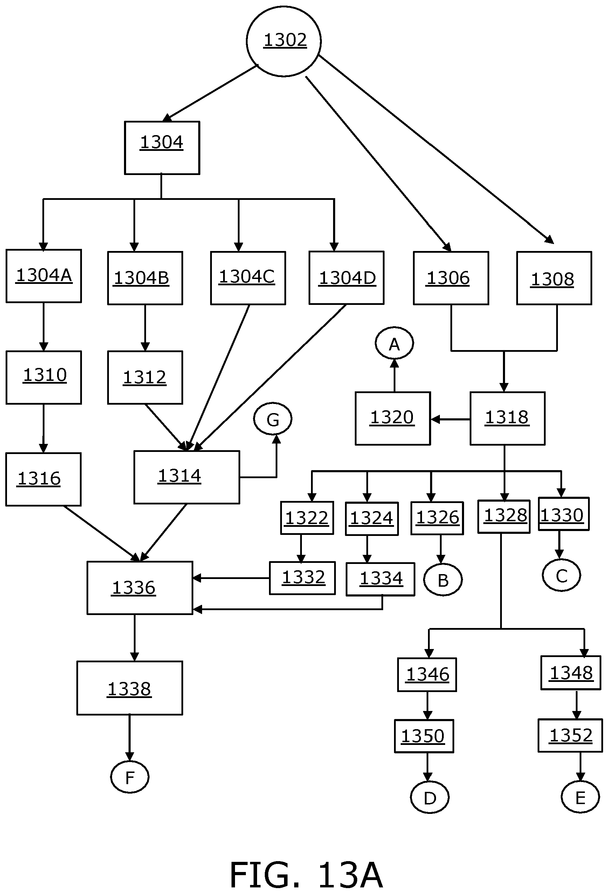

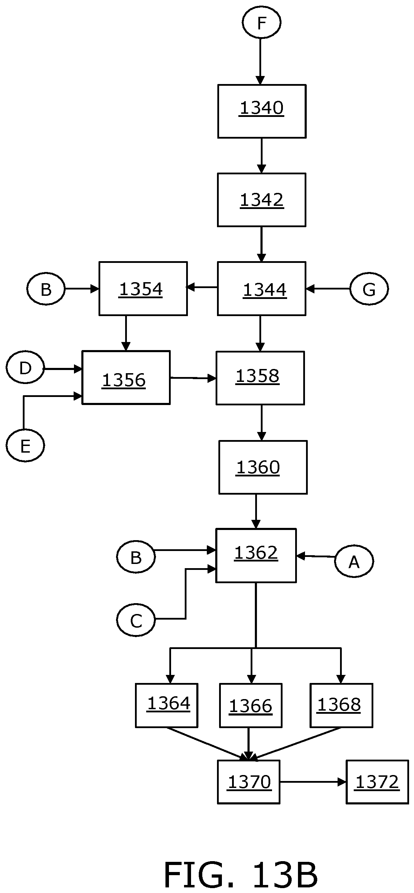

FIGS. 13A-13B are flow charts of an exemplary method for designing and manufacturing a scaled composite structure for protecting a body j oint, in accordance with an embodiment of the present disclosure. At a step **1302,** a user or a wearer provides at least one input parameter. At a step **1304,** data associated with the body joint to be protected is received. The data associated with the body joint to be protected includes a type of body joint **1304A,** an image of the body joint in a maximum range of motion or the maximum range of motion of the body joint as a manual input **1304B,** a length **1304C** of the scaled composite structure needed to cover the body joint, a width **1304D** of the scaled composite structure needed to cover the body j oint. The maximum range of motion represents a limit of the range of the motion to be applied to the scaled composite structure. At a step **1306,** body specifications of the wearer are received. At a step **1308,** a type of activity to be performed by the wearer is received. At a step **1310,** a range of motion of the body joint is determined based on the type of body joint **1304A.** The range of motion of the body joint may be a healthy range of the motion of the body joint. At a step **1312,** the maximum range of motion of the body joint is determined based on the image of the body joint in the maximum range of motion or the maximum range of motion of the body joint as the manual input **1304B.** At a step **1314,** a flexible base layer arrangement to be covered with a plurality of three dimensional (3D) scales is determined based on the maximum range of motion of the body joint, the length **1304C** of the scaled composite structure, and the width **1304D** of the scaled composite structure. At a step **1316,** a shape of a flexible base layer arrangement is determined based on the free range of motion of the body j oint. At a step **1318,** a force to be applied to the scaled composite structure is estimated based on the type of activity to be performed by the wearer and the body specifications of the wearer. At a step **1320,** a total weight of the scaled composite structure is determined based on estimated force to be applied to the scaled composite structure. At a step **1322,** a density of the scaled composite structure is determined based on the estimated force to be applied to the scaled composite structure. At a step **1324,** a base size of each of the plurality of 3D scales is determined based on the estimated force to be applied to the scaled composite structure. At a step **1326,** a thickness of each of the plurality of 3D scales is determined based on the estimated force to be applied to the scaled composite structure. At a step **1328,** a type of the flexible base layer arrangement to connect each of the plurality of 3D scales is selected based on the estimated force to be applied to the scaled composite structure. At a step **1330,** a type of material to manufacture the plurality of 3D scales is selected based on the estimated force to be applied to the scaled composite structure. At a step **1332,** a distance between each of two 3D scales within the scaled composite structure is determined based on the density of the scaled composite structure. At a step **1334,** an overall size of each of the plurality of 3D scales is determined based on the base size of each of the plurality of 3D scales. At a step **1336,** the flexible base layer arrangement is tessellated into the shape of the base unit based on the overall size of the each of the plurality of 3D scales and the distance between each of the plurality of 3D scales. At a step **1338,** each of the plurality of 3D scales is scaled to a size according to the shape of the flexible base layer arrangement and is arranged on the curved base layer according to a tessellation pattern. At a step **1340,** an intersection point between each two scales of the plurality of 3D scales in a row of 3D scales disposed on the flexible base layer arrangement when curved to the limit of the range of motion is determined. At a step **1342,** a length of each of the plurality of 3D scales in the flexible base layer arrangement is determined based on the intersection point between each two scales of the plurality of 3D scales. At a step **1344,** the flexible base layer arrangement is flattened that are arranged with the plurality of 3D scales. At a step **1346,** a degree of fineness of the flexible base layer arrangement is determined based on the type of the flexible base layer arrangement. At a step **1348,** a thickness of the flexible base layer arrangement is determined based on the type of the flexible base layer arrangement. At a step **1350,** a diameter of a plurality of teeth provided at a surface of the 3D scale facing towards the flexible base layer arrangement is determined based on the degree of fineness of the flexible base layer arrangement. At a step **1352,** a height of the plurality of teeth in each of the plurality of 3D scales is determined based on the thickness of the flexible base layer arrangement. At a step **1354,** a base layer of each of the plurality of 3D scales is extruded based on the flattened flexible base layer arrangement that is arranged with the plurality of 3D scales and the thickness of each of the plurality of 3D scales. At a step **1356,** the plurality of teeth is modelled based on extruded base layer of the each of the plurality of 3D scales, the diameter of the plurality of teeth in each of the plurality of 3D scales, and the height of the plurality of teeth in each of the plurality of 3D scales. At a step **1358,** a 3D model of the scaled composite structure is created based on the flattened flexible base layer arrangement that is arranged with the plurality of 3D scales and modelled plurality of teeth. At a step **1360,** the 3D model of the scaled composite structure is exported to a slicer of a 3D printer. At a step **1362,** the slicer is adjusted based on the 3D model of the scaled composite structure, the thickness of each of the plurality of 3D scales, the type of material to manufacture the plurality of 3D scales and the total weight of the scaled composite structure. At a step **1364,** a thickness of an extruder of the 3D printer is adjusted. At a step **1366,** a speed of the extruder of the 3D printer is adjusted. At a step **1368,** an infill structure and density are adjusted. At a step **1370,** a geometric code (GCODE) for 3D printing of the scaled composite structure is developed based on settings of the 3D printer. At a step **1372,** the scaled composite structure is manufactured based on developed GCODE.

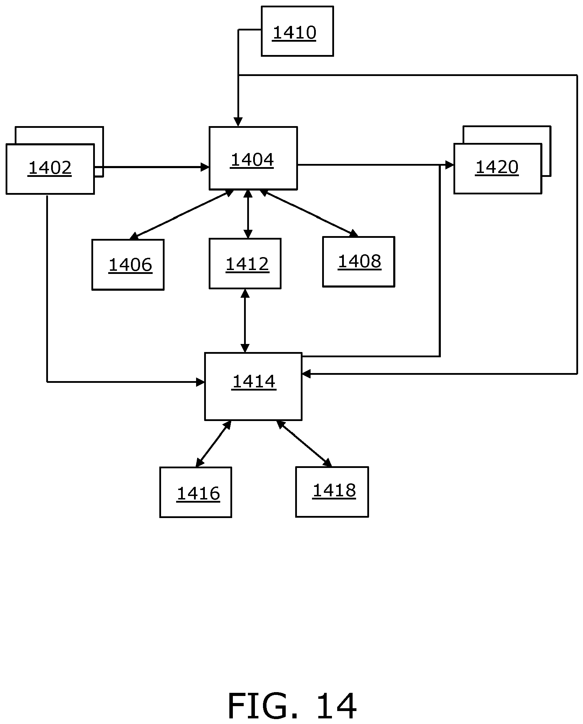

FIG. 14 is an illustration of an exploded view of a distributed computing architecture or a system in accordance with an embodiment of the present disclosure. The exploded view comprises a user device or a client device that comprises an input interface **1402,** a control module that comprises a processor **1404,** a memory **1406** and a non-volatile storage **1408,** processing instructions **1410,** a shared or distributed storage **1412,** a server that comprises a server processor **1414,** a server memory **1416** and a server non-volatile storage **1418** and an output interface **1420.** The function of the processor **1404,** the memory **1406** and the non-volatile storage **1408** are thus identical to the server processor **1414,** the server memory **1416** and the server non-volatile storage **1418** respectively. The functions of these parts are as described above.

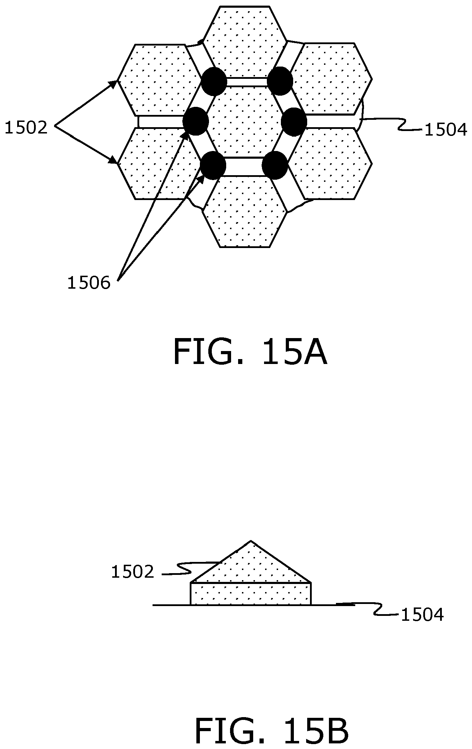

FIG. 15A and 15B are perspective views of the plurality of 3D scales **1502** connected through a base plate **1504** in accordance with an embodiment of the present disclosure. FIG. 15A shows a top view of the plurality of 3D scales **1502** connected together using the base plate **1504** comprising a plurality of holes **1506.** FIG. 15B show a side view of the plurality of 3D scales **1502** connected together using a base plate **1504.**

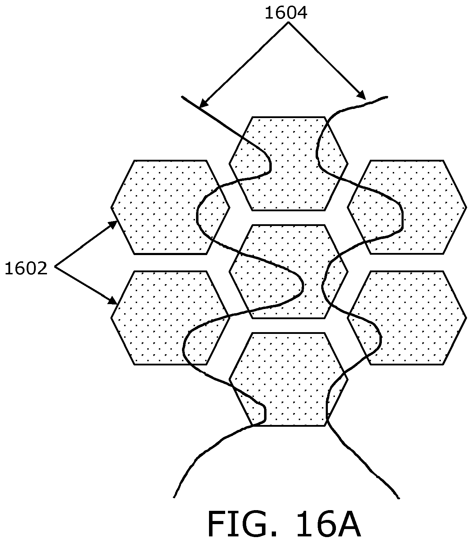

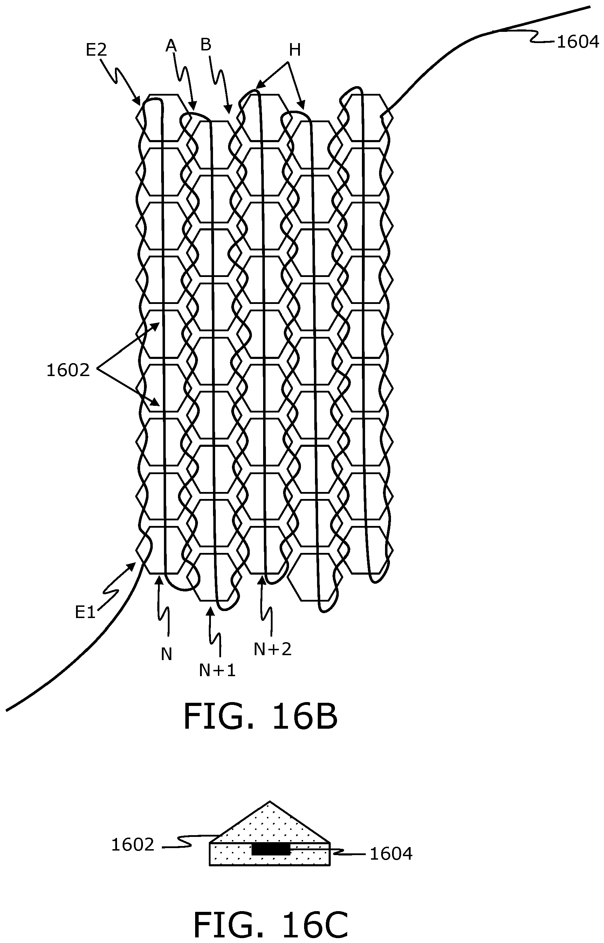

FIG. 16A, 16B and 16C are perspective views of the plurality of 3D scales **1602** connected through stitches **1604** in accordance with an embodiment of the present disclosure. FIG. 16A shows a top view of the plurality of 3D scales **1602** connected together using the stitches **1604,** such as Kevlar stiches. As shown, the Kevlar stitches are used to bond a base unit of a 3D scale **1602** and a top part of an adjacent 3D scale **1602.**

FIG 16B illustrates the plurality of 3D scales **1602** connected through a single length of Kevlar stitch **1604.** As shown, in a first stitch **A,** the Kevlar stitch **1604** connects the adjacent 3D scales **1602,** arranged in a $N^{th}$ row and a $N^{th}+1$ row parallel and adjacent to the $N^{th}$ row, from a first end **E1** of each of said parallel rows to a second end **E2** of each of said parallel rows. As shown, from the second end **E2** of the $N^{th}+1$ row, the Kevlar stitch **1604** runs centrally through the 3D scales **1602** arranged in the $N^{th}+1$ row, making a hair-pin loop **H** like structure at the second end **E2** of the $N^{th}+1$ row.

Similarly, in a second stitch **B,** the Kevlar stitch **1604** connects the adjacent 3D scales **1602,** arranged in $N^{th}+1$ row and a $N^{th}+2$ row parallel and adjacent to the $N^{th}+1$ row, from a first end **E1** of each of said parallel rows to a second end **E2** of each of said parallel rows. As shown, from the second end **E2** of the $N^{th}+2$ row, the Kevlar stitch **1604** runs centrally through the 3D scales **1602** arranged in the N+2 row, making a hair-pin loop **H** like structure at the second end **E2** of the $N^{th}+2$ parallel row.

FIG. 16C shows a side view of the plurality of 3D scales **1602** connected together using Kevlar stiches **1604** running in between the adjacent 3D scales **1602.**

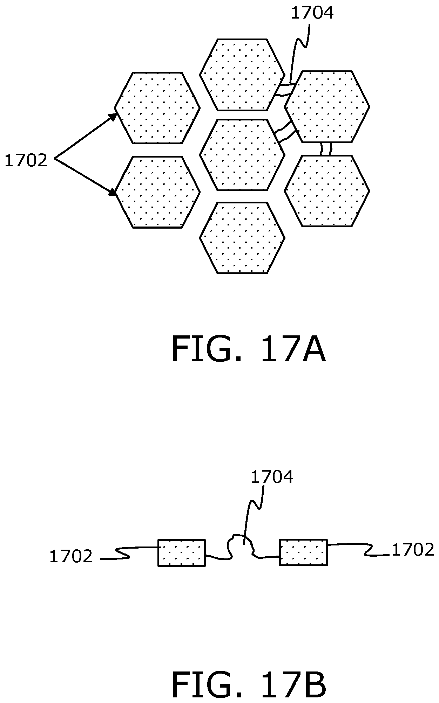

FIG. 17A and 17B are perspective views of the plurality of 3D scales **1702** connected through living hinges **1704** in accordance with an embodiment of the present disclosure. FIG. 17A shows top view of the plurality of 3D scales **1702** connected together using the living hinges **1704.** As shown, connecting the plurality of 3D scales **1702** connected together using the living hinges **1704** does not require a base plate, such as the base plate **1504** of FIG 15A. FIG. 17B show a side view of the plurality of 3D scales **1702** connected together using living hinges **1704.**

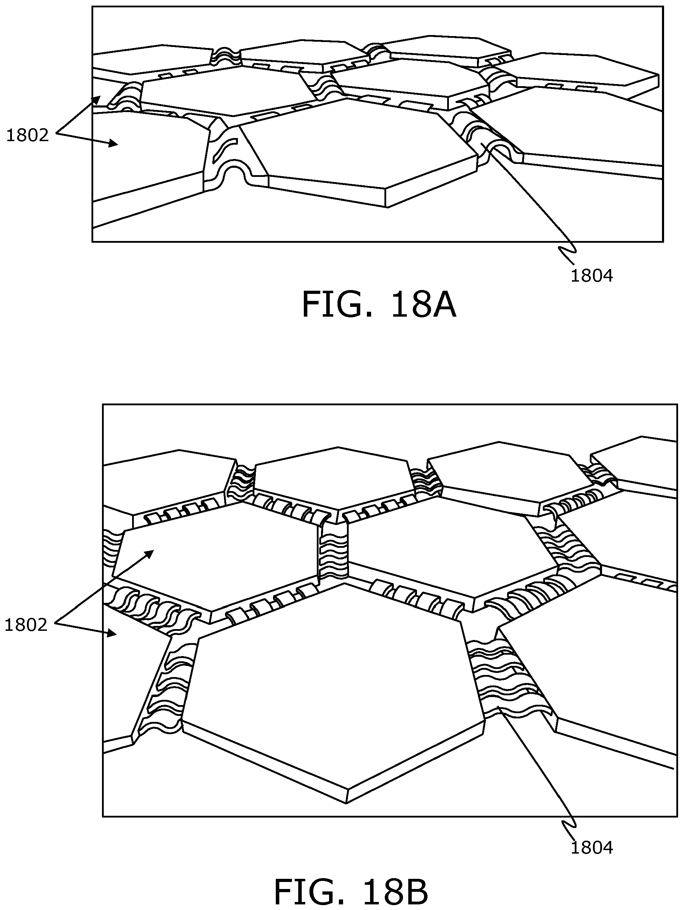

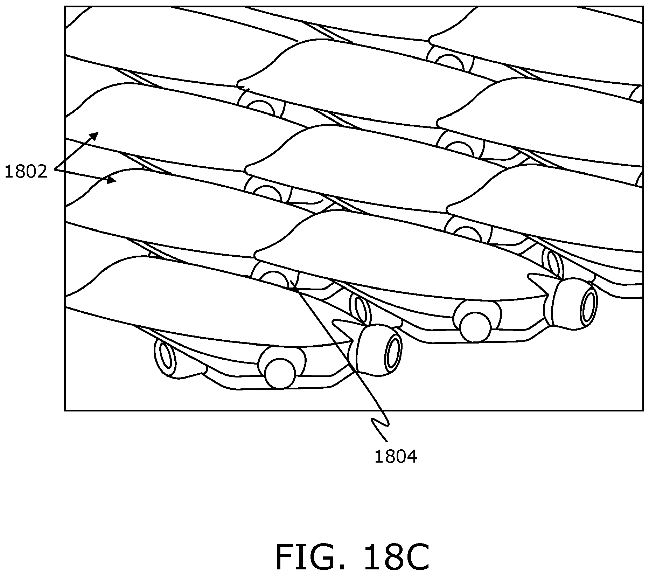

FIG. 18A, 18B and 18C are illustrations of exemplary living hinges **1802** for connecting plurality of 3D scales **1804** in accordance with an embodiment of the present disclosure. As shown in FIG. 18A, the adjacent 3D scales **1804** are connected together using a pair of living hinges **1802.** As shown in FIG. 18B, the adjacent 3D scales **1804** are connected together using four living hinges **1802.** As shown in FIG. 18C, the adjacent 3D scales **1804** are connected together using a single living hinge **1802.**

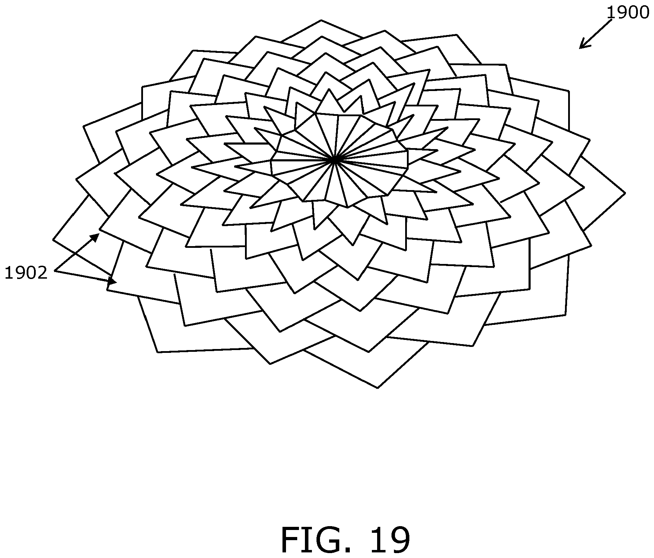

FIG. 19 is an illustration of an exemplary scaled composite structure **1900** in accordance with various embodiments of the present disclosure. As shown, the plurality of 3D scales **1902** are radially arranged to result in a high strength, flexible scaled composite structure **1900.**

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

**Claims**

1. A scaled composite structure (200) comprising:

    (i) a flexible base layer arrangement (204); and
    (ii) a plurality of three-dimensional (3D) scales (202A-N) attached to the flexible base layer arrangement (204), wherein the plurality of 3D scales (202A-N) are overlapping when the scaled composite structure (200) is placed on a planar surface,

    wherein a range of motion of the scaled composite structure (200) is controlled through a size and a shape of each of the plurality of 3D scales (202A-N) such that the plurality of 3D scales (202A-N) are intersecting with each other when a limit of the range of motion is applied to the scaled composite structure (200), to provide a mechanical interlocking effect,
    **characterised in that**
    an intersection point between each two scales of the plurality of 3D scales (202A-N) in a row of scales disposed on the flexible base layer arrangement (204) when curved to the limit of the range of motion determines the length of the of each of the plurality of 3D scales (202A-N) in the flexible base layer arrangement (204) when curved to the limit of the range of motion.

2. A scaled composite structure (200) according to claim 1, wherein the flexible base layer arrangement (204) enables each of the plurality of 3D scales (202A-N) to only rotate around a base plane in a centre point of each of the plurality of 3D scales (202A-N).

3. A scaled composite structure according to claim 1 or 2, wherein the flexible base layer arrangement (204) operates against a force that is produced when the limit of the range of motion is applied to the scaled composite structure (200) until interlocking of each of the plurality of 3D scales (202A-N) such that the scaled composite structure (200) provides impact protection against produced force through force distribution.

4. A scaled composite structure (200) according to any one of claims 1 to 3, wherein the size and the shape of at least a given scale of the plurality of 3D scales (202A-N) is a function of a location of the given scale within the scaled composite structure (200).

5. A scaled composite structure (200) according to any one of claims 1 to 4, wherein a length and a width of the each of the plurality of 3D scales (202A-N) are in a range of 0.01 millimetres (mm) to 500 mm.

6. A scaled composite structure (200) according to any one of claims 1 to 5, wherein each of the plurality of 3D scales (202A-N) comprises a body portion (302) and a nose portion (304), wherein the nose portion (304) is characterized as a front extension of each of the plurality of 3D scales (202A-N) which overlaps a preceding 3D scale in the scaled composite structure (200).

7. A wearable protective device (800) comprising:

    (i) a flexible base layer arrangement (804); and
    (ii) a plurality of three-dimensional (3D) scales (802A-N) attached to the flexible base layer arrangement (804), wherein the plurality of 3D scales (802A-N) are overlapping when the wearable protective device is placed on a planar surface;

    wherein a range of motion of the wearable protective device (800) is controlled through a size and a shape of each of the plurality of 3D scales (802A-N) such that the plurality of 3D scales (802A-N) are intersecting with each other when a limit of the range of motion is applied to the wearable protective device (800), to provide a mechanical interlocking effect,
    **characterised in that**
    the intersection point between each two scales of the plurality of 3D scales (802A-N) in a row of scales disposed on the flexible base layer arrangement (804) when curved to the limit of the range of motion determines the length of the each of the plurality of 3D scales (802A-N) in the flexible base layer arrangement (804) when curved to the limit of the range of motion.

8. A wearable protective device (800) according to claim 7, wherein the wearable protective device (800) further comprises at least one of a double-sided adhesive layer, a type of hydrogel adhesive layer, a silicone adhesive layer or a rubber adhesive layer on one side of the wearable protective device (800) or a sleeve that is interlaced with the wearable protective device (800), for attaching to a skin of a wearer.

9. A method for designing and manufacturing a scaled composite structure, wherein the scaled composite structure comprises a plurality of three-dimensional (3D) scales that are attached to a flexible base layer arrangement, wherein the method comprises:

   determining, by using a data processing arrangement, (1002), a flexible base layer arrangement and a shape of the flexible base layer arrangement, based on at least one input parameter, at a limit of a range of motion to be applied to the scaled composite structure;
   determining, by using the data processing arrangement, (1004) a size and a shape of each of the plurality of 3D scales that are arranged on the flexible base layer arrangement when curved to the limit of the range of motion, thereby determining a length of each of the plurality of 3D scales; and
   manufacturing the scaled composite structure based on the size and the shape of each of the plurality of 3D scales that are arranged on the flexible base layer arrangement when curved to the limit of the range of motion, (1006), such that the scaled composite structure provides a mechanical interlocking effect when the limit of the range of motion is applied to the scaled composite structure and is flexible until each of the plurality of 3D scales interlock with each other,
   **characterised in that**
   the method further comprises determining an intersection point between each two scales of the plurality of 3D scales in a row of scales disposed on the flexible base layer arrangement when curved to the limit of the range of motion, to determine the length of the of each of the plurality of 3D scales in the flexible base layer arrangement when curved to the limit of the range of motion.

10. A method according to claim 9, further comprising

   determining, by using the data processing arrangement, (1334), an overall size of each of the plurality of 3D scales and a distance between each of the plurality of 3D scales, based on the at least one input parameter;
   tessellating, by using the data processing arrangement, (1336), the flexible base layer arrangement when curved to the limit of the range of motion based on the overall size of the each of the plurality of 3D scales and the distance between each of the plurality of 3D scales; and arranging, (1338), by using the data processing arrangement, the plurality of 3D scales on the flexible base layer arrangement according to a size and the shape of a base unit after tessellating the flexible base layer arrangement when curved to the limit of the range of motion to determine, (1340), the size and the shape of each of the plurality of 3D scales.

11. A method according to claim 10, further comprising estimating, (1318), a force to be applied to the scaled composite structure based on the at least one input parameter for enabling determining the overall size of each of the plurality of 3D scales and the distance between each of the plurality of 3D scales.

12. A method according to claim 11, further comprising determining (i) a thickness of each of the plurality of 3D scales, (1326); (ii) a type of the flexible base layer arrangement for connecting each of the plurality of 3D scales, (1328); and (iii) a material for manufacturing the plurality of 3D scales, based on an estimated force, (1330).

13. A method according to any one of claims 9 to 12, wherein the size and the shape of at least one a given scale of the plurality of 3D scales are a function of a location of the given scale within the scaled composite structure, wherein the mechanical interlocking effect of the scaled composite structure is controlled through the size and the shape of each of the plurality of 3D scales.

14. A method according to any one of claims 9 to 13, wherein the manufacturing of the scaled composite structure comprises:

   - providing a flexible base layer arrangement;

- generating a bottom layer of at least one scale of the plurality of 3D scales;
- generating a plurality of teeth like structures projecting from the bottom layer;
- adding a first layer above the bottom layer; and
- generating a top layer of the at least one scale of the plurality of 3D scales on top of the first layer.

**15.** A computer program product comprising instructions which allow a system (100) comprising a memory (102), a data processing arrangement (104) and a scale forming unit (106) to carry out the method of any one of claims 9 to 14.

**Patentansprüche**

**1.** Skalierte Verbundstruktur (200), umfassend:

(i) eine flexible Basisschichtanordnung (204); und
(ii) eine Vielzahl von dreidimensionalen (3D) Skalen (202A-N), die an der flexiblen Basisschichtanordnung (204) angebracht sind, wobei sich die Vielzahl von 3D-Skalen (202A-N) überlappen, wenn die skalierte Verbundstruktur (200) auf einer planaren Oberfläche platziert wird,

wobei ein Bewegungsbereich der skalierten Verbundstruktur (200) durch eine Größe und eine Form jeder der Vielzahl von 3D-Skalen (202A-N) gesteuert wird, sodass die Vielzahl von 3D-Skalen (202A-N) einander schneiden, wenn eine Grenze des Bewegungsbereichs auf die skalierte Verbundstruktur (200) angewendet wird, um einen mechanischen Verriegelungseffekt zu erzielen,
**dadurch gekennzeichnet, dass** ein Schnittpunkt zwischen jeweils zwei Skalen der Vielzahl von 3D-Skalen (202A-N) in einer Reihe von Skalen, die auf der flexiblen Basisschichtanordnung (204) angeordnet sind, bei einer Biegung bis zu der Grenze des Bewegungsbereichs die Länge jeder der Vielzahl von 3D-Skalen (202A-N) in der flexiblen Basisschichtanordnung (204) bei einer Biegung bis zu der Grenze des Bewegungsbereichs bestimmt.

**2.** Skalierte Verbundstruktur (200) nach Anspruch 1, wobei die flexible Basisschichtanordnung (204) jeder der Vielzahl von 3D-Skalen (202A-N) ermöglicht, sich nur um eine Basisebene in einem Mittelpunkt jeder der Vielzahl von 3D-Skalen (202A-N) zu drehen.

**3.** Skalierte Verbundstruktur nach Anspruch 1 oder 2, wobei die flexible Basisschichtanordnung (204) gegen eine Kraft wirkt, die erzeugt wird, wenn die Grenze des Bewegungsbereichs auf die skalierte Verbundstruktur (200) angewendet wird, bis jede der Vielzahl von 3D-Skalen (202A-N) derart ineinandergreift, dass die skalierte Verbundstruktur (200) durch Kraftverteilung einen Aufprallschutz gegen die erzeugte Kraft bereitstellt.

**4.** Skalierte Verbundstruktur (200) nach einem der Ansprüche 1 bis 3, wobei die Größe und die Form wenigstens einer gegebenen Skala der Vielzahl von 3D-Skalen (202A-N) eine Funktion einer Position der gegebenen Skala innerhalb der skalierten Verbundstruktur (200) ist.

**5.** Skalierte Verbundstruktur (200) nach einem der Ansprüche 1 bis 4, wobei eine Länge und eine Breite jeder der Vielzahl von 3D-Skalen (202A-N) in einem Bereich von 0,01 Millimeter (mm) bis 500 mm liegt.

**6.** Skalierte Verbundstruktur (200) nach einem der Ansprüche 1 bis 5, wobei jede der Vielzahl von 3D-Skalen (202A-N) einen Körperabschnitt (302) und einen Nasenabschnitt (304) aufweist, wobei der Nasenabschnitt (304) als eine vordere Verlängerung jeder der Vielzahl von 3D-Skalen (202A-N) gekennzeichnet ist, die eine vorhergehende 3D-Skala in der skalierten Verbundstruktur (200) überlappt.

**7.** Tragbare Schutzvorrichtung (800), umfassend:

(i) eine flexible Basisschichtanordnung (804); und
(ii) eine Vielzahl von dreidimensionalen (3D) Skalen (802A-N), die an der flexiblen Basisschichtanordnung (804) angebracht sind, wobei sich die Vielzahl von 3D-Skalen (802A-N) überlappen, wenn die tragbare Schutzvorrichtung auf einer planaren Oberfläche platziert wird;

wobei ein Bewegungsbereich der tragbaren

Schutzvorrichtung (800) durch eine Größe und eine Form jeder der Vielzahl von 3D-Skalen (802A-N) gesteuert wird, sodass sich die Vielzahl von 3D-Skalen (802A-N) gegenseitig überschneiden, wenn eine Grenze des Bewegungsbereichs auf die tragbare Schutzvorrichtung (800) angewendet wird, um einen mechanischen Verriegelungseffekt zu erzielen,

**dadurch gekennzeichnet, dass** der Schnittpunkt zwischen jeweils zwei Skalen der Vielzahl von 3D-Skalen (802A-N) in einer Reihe von Skalen, die auf der flexiblen Basisschichtanordnung (804) angeordnet sind, bei Biegung bis zu der Grenze des Bewegungsbereichs, die Länge jeder der Vielzahl von 3D-Skalen (802A-N) in der flexiblen Basisschichtanordnung (804) bei Biegung bis zu der Grenze des Bewegungsbereichs bestimmt.

8. Tragbare Schutzvorrichtung (800) nach Anspruch 7, wobei die tragbare Schutzvorrichtung (800) ferner wenigstens eine doppelseitige Klebeschicht, eine Art Hydrogel-Klebeschicht, eine Silikon-Klebeschicht oder eine Gummi-Klebeschicht auf einer Seite der tragbaren Schutzvorrichtung (800) oder eine Manschette, die mit der tragbaren Schutzvorrichtung (800) verflochten ist, zum Befestigen an der Haut eines Trägers, umfasst.

9. Verfahren zum Entwerfen und Herstellen einer skalierten Verbundstruktur, wobei die skalierte Verbundstruktur eine Vielzahl von dreidimensionalen (3D) Skalen umfasst, die an einer flexiblen Basisschichtanordnung angebracht sind, wobei das Verfahren Folgendes umfasst:

Bestimmen, unter Verwendung einer Datenverarbeitungsanordnung (1002), einer flexiblen Basisschichtanordnung und einer Form der flexiblen Basisschichtanordnung, basierend auf wenigstens einem Eingabeparameter, an einer Grenze eines Bewegungsbereichs, der auf die skalierte Verbundstruktur anzuwenden ist; Bestimmen, unter Verwendung der Datenverarbeitungsanordnung (1004), einer Größe und einer Form von jeder der Vielzahl von 3D-Skalen, die auf der flexiblen Basisschichtanordnung angeordnet sind, bei einer Biegung bis zu der Grenze des Bewegungsbereichs, wodurch eine Länge von jeder der Vielzahl von 3D-Skalen bestimmt wird; und Herstellen der skalierten Verbundstruktur basierend auf der Größe und der Form jeder der Vielzahl von 3D-Skalen, die auf der flexiblen Basisschichtanordnung angeordnet sind, bei Biegung bis zu der Grenze des Bewegungsbereichs (1006), sodass die skalierte Verbundstruktur einen mechanischen Verriegelungseffekt bereitstellt, wenn die Grenze des Bewegungsbereichs auf die skalierte Verbundstruktur angewendet wird, und flexibel ist, bis jede der Vielzahl von 3D-Skalen miteinander verriegelt ist, **dadurch gekennzeichnet, dass** das Verfahren ferner das Bestimmen eines Schnittpunkts zwischen jeweils zwei Skalen der Vielzahl von 3D-Skalen in einer Reihe von Skalen, die auf der flexiblen Basisschichtanordnung angeordnet sind, bei Biegung bis zu der Grenze des Bewegungsbereichs zum Bestimmen der Länge jeder der Vielzahl von 3D-Skalen in der flexiblen Basisschichtanordnung bei Biegung bis zu der Grenze des Bewegungsbereichs umfasst.

10. Verfahren nach Anspruch 9, ferner umfassend: Bestimmen, unter Verwendung der Datenverarbeitungsanordnung (1334), einer Gesamtgröße jeder der Vielzahl von 3D-Skalen und eines Abstandes zwischen jeder der Vielzahl von 3D-Skalen, basierend auf dem wenigstens einen Eingabeparameter; Tessellieren, unter Verwendung der Datenverarbeitungsanordnung (1336), der flexiblen Basisschichtanordnung bei Biegung bis zu der Grenze des Bewegungsbereichs basierend auf der Gesamtgröße jeder der Vielzahl von 3D-Skalen und dem Abstand zwischen jeder der Vielzahl von 3D-Skalen; und Anordnen (1338), unter Verwendung der Datenverarbeitungsanordnung, der Vielzahl von 3D-Skalen auf der flexiblen Basisschichtanordnung gemäß einer Größe und der Form einer Basiseinheit nach dem Tessellieren der flexiblen Basisschichtanordnung bei Biegung bis zu der Grenze des Bewegungsbereichs, um die Größe und die Form jeder der Vielzahl von 3D-Skalen zu bestimmen (1340).

11. Verfahren nach Anspruch 10, ferner umfassend das Schätzen (1318) einer auf die skalierte Verbundstruktur aufzubringenden Kraft basierend auf dem wenigstens einen Eingabeparameter, um die Bestimmung der Gesamtgröße jeder der Vielzahl von 3D-Skalen und des Abstands zwischen jeder der Vielzahl von 3D-Skalen zu ermöglichen.

12. Verfahren nach Anspruch 11, ferner umfassend das Bestimmen (i) einer Dicke jeder der Vielzahl von 3D-Skalen (1326); (ii) einer Art der flexiblen Basisschichtanordnung zum Verbinden jeder der Vielzahl von 3D-Skalen (1328); und (iii) eines Materials zum Herstellen der Vielzahl von 3D-Skalen, basierend auf einer geschätzten Kraft (1330).

13. Verfahren nach einem der Ansprüche 9 bis 12, wobei die Größe und die Form wenigstens einer gegebenen Skala der Vielzahl von 3D-Skalen eine Funktion einer Position der gegebenen Skala innerhalb der skalierten Verbundstruktur ist, wobei der mechanische Verriegelungseffekt der skalierten Verbundstruktur durch die Größe und die Form jeder der Vielzahl von 3D-Skalen gesteuert wird.

14. Verfahren nach einem der Ansprüche 9 bis 13, wobei die Herstellung der skalierten Verbundstruktur Folgendes umfasst:

- Bereitstellen einer flexiblen Basisschichtanordnung;
- Erzeugen einer unteren Schicht von wenigstens einer Skala der Vielzahl von 3D-Skalen;
- Erzeugen einer Vielzahl von zahnähnlichen Strukturen, die aus der unteren Schicht vorstehen;
- Hinzufügen einer ersten Schicht über der unteren Schicht; und
- Erzeugen einer oberen Schicht der wenigstens einen Skala der Vielzahl von 3D-Skalen auf der ersten Schicht.

15. Computerprogrammprodukt, umfassend Anweisungen, die einem System (100), das einen Speicher (102), eine Datenverarbeitungsanordnung (104) und eine Skalenbildungseinheit (106) umfasst, das Ausführen des Verfahrens nach einem der Ansprüche 9 bis 14 ermöglichen.

**Revendications**

1. Structure composite mise à l'échelle (200) comprenant :

(i) un agencement de couche de base flexible (204) ; et
(ii) une pluralité d'échelles tridimensionnelles (3D) (202A-N) fixées à l'agencement de couche de base flexible (204), dans lequel la pluralité d'échelles 3D (202A-N) se chevauche lorsque la structure composite mise à l'échelle (200) est placée sur une surface plane,

dans lequel une plage de mouvement de la structure composite mise à l'échelle (200) est commandée à travers une taille et une forme de chacune des échelles 3D (202A-N) de telle sorte que la pluralité d'échelles 3D (202A-N) se croisent les unes les autres lorsqu'une limite de la plage de mouvement est appliquée à la structure composite mise à l'échelle (200), pour fournir un effet de verrouillage mécanique,
**caractérisé en ce qu'**un point d'intersection entre chaque paire d'échelles de la pluralité d'échelles 3D (202A-N) dans une rangée d'échelles disposées sur l'agencement de couche de base flexible (204) lorsqu'elles sont courbées à la limite de la plage de mouvement détermine la longueur de chacune de la pluralité d'échelles 3D (202A-N) dans l'agencement de couche de base flexible (204) lorsqu'elles sont courbées à la limite de la plage de mouvement.

2. Structure composite mise à l'échelle (200) selon la revendication 1, dans laquelle l'agencement de couche de base flexible (204) permet à chacune de la pluralité d'échelles 3D (202A-N) de ne tourner qu'autour d'un plan de base en un point central de chacune de la pluralité d'échelles 3D (202A-N).

3. Structure composite mise à l'échelle selon la revendication 1 ou 2, dans laquelle l'agencement de couche de base flexible (204) agit contre une force qui est produite lorsque la limite de la plage de mouvement est appliquée à la structure composite mise à l'échelle (200) jusqu'à l'emboîtement de chacune de la pluralité d'échelles 3D (202AN) de telle sorte que la structure composite mise à l'échelle (200) assure une protection antichocs contre les forces produites par l'intermédiaire de la répartition des forces.

4. Structure composite mise à l'échelle (200) selon l'une quelconque des revendications 1 à 3, dans laquelle la taille et la forme de l'au moins une échelle donnée de la pluralité d'échelles 3D (202A-N) est une fonction d'un emplacement de l'échelle donnée à l'intérieur de la structure composite mise à l'échelle (200).

5. Structure composite mise à l'échelle (200) selon l'une quelconque des revendications 1 à 4, dans laquelle une longueur et une largeur de chacune de la pluralité d'échelles 3D (202A-N) sont dans une plage de 0,01 millimètre (mm) à 500 mm.

6. Structure composite mise à l'échelle (200) selon l'une quelconque des revendications 1 à 5, dans laquelle chacune de la pluralité d'échelles 3D (202A-N) comprend une partie de corps (302) et une partie de nez (304), dans laquelle la partie de nez (304) est **caractérisée** comme une extension avant de chacune de la pluralité d'échelles 3D (202A-N) qui chevauche une échelle 3D précédente dans la structure composite mise à l'échelle (200).

7. Dispositif de protection portable (800) comprenant :

(i) un agencement de couche de base flexible (804) ; et
(ii) une pluralité d'échelles tridimensionnelles (3D) (802A-N) fixées à l'agencement de couche de base flexible (804), dans lequel la pluralité d'échelles 3D (802A-N) se chevauche lorsque le dispositif protecteur portable est placé sur une surface plane ;

dans lequel une plage de mouvement du dispositif de protection portable (800) est commandée à travers une taille et une forme de chacune des échelles 3D (802A-N) de telle sorte que la pluralité d'échelles 3D (802A-N) se coupent les unes les autres lorsqu'une limite de la plage de mouvement est appliquée au dispositif de protection portable (800), pour fournir un effet de verrouillage mécanique, **caractérisé en ce que** le point d'intersection entre chaque paire d'échelles de la pluralité d'échelles 3D (802A-N) dans une rangée d'échelles disposées sur l'agencement de couche de base flexible (804) lorsqu'elles sont courbées à la limite de la plage de mouvement détermine la longueur de chacune de la pluralité d'échelles 3D (802A-N) dans l'agencement de couche de base flexible (804) lorsqu'elles sont courbées à la limite de la plage de mouvement.

8. Dispositif de protection portable (800) selon la revendication 7, dans lequel le dispositif de protection portable (800) comprend en outre l'au moins une couche adhésive double face, un type de couche adhésive d'hydrogel, une couche adhésive de silicone ou une couche adhésive de caoutchouc sur un côté du dispositif de protection portable (800) ou un manchon qui est entrelacé avec le dispositif de protection portable (800), pour être fixé à la peau d'un utilisateur.

9. Procédé de conception et de fabrication d'une structure composite mise à l'échelle, dans lequel la structure composite mise à l'échelle comprend une pluralité d'échelles tridimensionnelles (3D) qui sont fixées à un agencement de couche de base flexible, dans lequel le procédé comprend :

la détermination, en utilisant un agencement de traitement de données, (1002), un agencement de couche de base flexible et une forme de l'agencement de couche de base flexible, sur la base de l'au moins un paramètre d'entrée, à une limite d'une plage de mouvement à appliquer à la structure composite mise à l'échelle ; la détermination, en utilisant le dispositif de traitement de données, (1004) une taille et une forme de chacune de la pluralité d'échelles 3D qui sont disposées sur l'agencement de couche de base flexible lorsqu'elles sont courbées à la limite de la plage de mouvement, déterminer ainsi une longueur de chacune de la pluralité d'échelles 3D ; et
la fabrication de la structure composite mise à l'échelle sur la base de la taille et de la forme de chacune des échelles 3D qui sont agencées sur l'agencement de couche de base flexible lorsqu'elles sont courbées à la limite de la plage de mouvement (1006), de sorte que la structure composite mise à l'échelle fournit un effet de verrouillage mécanique lorsque la limite de la plage de mouvement est appliquée à la structure composite mise à l'échelle et est flexible jusqu'à ce que chacune de la pluralité d'échelle 3D se verrouille l'une avec l'autre, **caractérisé en ce que** le procédé comprend en outre la détermination d'un point d'intersection entre chaque paire d'échelles de la pluralité d'échelles 3D dans une rangée d'échelles disposées sur l'agencement de couche de base flexible lorsqu'elles sont courbées à la limite de la plage de mouvement, pour déterminer la longueur de chacune de la pluralité d'échelles 3D dans l'agencement de couche de base flexible lorsqu'elles sont courbées à la limite de la plage de mouvement.

10. Procédé selon la revendication 9, comprenant en outre la détermination, en utilisant le dispositif de traitement de données, (1334), d'une taille globale de chacune des échelles 3D et une distance entre chacune des échelles 3D, sur la base de l'au moins un des paramètres d'entrée ; tessellation, en utilisant le dispositif de traitement de données, (1336), de l'agencement de couche de base flexible lorsqu'il est courbé à la limite de la plage de mouvement sur la base de la taille globale de chacune des échelles 3D et de la distance entre chacune des échelles 3D ; et l'agencement, (1338), en utilisant le dispositif de traitement de données, la pluralité d'échelles 3D sur l'agencement de couche de base flexible selon une taille et la forme d'une unité de base après avoir disséminé l'agencement de couche de base flexible lorsqu'il est incurvé à la limite de la plage de mouvement pour déterminer, (1340), la taille et la forme de chacune de la pluralité d'échelles 3D.

11. Procédé selon la revendication 10, comprenant en outre l'estimation (1318) d'une force à appliquer à la structure composite mise à l'échelle sur la base d'au moins un des paramètres d'entrée pour permettre de déterminer la taille globale de chacune des échelles 3D et la distance entre chacune d'entre elles.

**12.** Procédé selon la revendication 11, comprenant en outre la détermination (i) d'une épaisseur de chacune des échelles 3D (1326) ; (ii) d'un type de l'agencement de couche de base flexible pour connecter chacune des échelles 3D (1328) ; et (iii) d'un matériau pour fabriquer la pluralité d'échelles 3D, sur la base d'une force estimée (1330).

**13.** Procédé selon l'une quelconque des revendications 9 à 12, dans lequel la taille et la forme d'au moins une échelle donnée de la pluralité d'échelles 3D sont une fonction d'un emplacement de l'échelle donnée à l'intérieur de la structure composite mise à l'échelle, dans lequel l'effet de verrouillage mécanique de la structure composite mise à l'échelle est commandé par la taille et la forme de chacune des échelles 3D.

**14.** Procédé selon l'une quelconque des revendications 9 à 13, dans lequel la fabrication de la structure composite mise à l'échelle comprend :

- la fourniture d'un agencement de couche de base flexible ;
- la génération d'une couche inférieure de l'au moins une échelle de la pluralité d'échelles 3D ;
- la génération d'une pluralité de structures de type dents faisant saillie à partir de la couche inférieure ;
- l'ajout d'une première couche au-dessus de la couche inférieure ; et
- la génération d'une couche supérieure de l'au moins une échelle de la pluralité d'échelles 3D sur le dessus de la première couche.

**15.** Produit de programme informatique comprenant des instructions qui permettent à un système (100) comprenant une mémoire (102), un agencement de traitement de données (104) et une unité de formation d'échelle (106) d'exécuter le procédé selon l'une quelconque des revendications 9 à 14.

FIG. 1

200

202A

202B

202N

210

204

206

FIG. 2A

210

204

208

206

# FIG. 2B

300A

304

302

# FIG. 3A

300B

308

306

# FIG. 3B

300C

312

310

# FIG. 3C

400

404A

402A

404N

404B

402B

# FIG. 4

502

504

506

# FIG. 5A

510

X

508

512

# FIG. 5B

602N

602B

602A

600

610

608

606

604

FIG. 6

FIG. 7A

FIG. 7B

FIG. 8A

810

804

808

806

FIG. 8B

FIG. 9

DETERMINING, BY USING A DATA PROCESSING ARRANGEMENT, A FLEXIBLE BASE LAYER ARRANGEMENT AND A SHAPE OF THE FLEXIBLE BASE LAYER ARRANGEMENT, BASED ON AT LEAST ONE INPUT PARAMETER

1002

DETERMINING, BY USING THE DATA PROCESSING ARRANGEMENT, A SIZE AND A SHAPE OF EACH OF THE PLURALITY OF 3D SCALES THAT ARE ARRANGED ON THE FLEXIBLE BASE LAYER ARRANGEMENT WHEN CURVED TO THE LIMIT OF THE RANGE OF MOTION,THEREBY DETERMINING A LENGTH OF EACH OF THE PLURALITY OF 3D SCALES

1004

MANUFACTURING THE SCALED COMPOSITE STRUCTURE BASED ON THE SIZE AND THE SHAPE OF EACH OF THE PLURALITY OF 3D SCALES THAT ARE ARRANGED ON THE FLEXIBLE BASE LAYER ARRANGEMENT WHEN CURVED TO THE LIMIT OF THE RANGE OF MOTION

1006

FIG. 10

FIG. 11

FIG. 12

FIG. 13A

FIG. 13B

1410

1402

1404

1420

1406

1412

1408

1414

1416

1418

FIG. 14

1502

1504

1506

# FIG. 15A

1502

1504

# FIG. 15B

FIG. 16A

FIG. 16B

FIG. 16C

FIG. 17A

FIG. 17B

FIG. 18A

FIG. 18B

FIG. 18C

FIG. 19

**EP 4 247 201 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009276943 A1
- US 3867239 A
- WO 0187432 A2
- WO 2018224845 A1